(19) Europäisches Patentamt
European Patent Office
Office européen des brevets

(11) EP 1 015 609 B1

(12) EUROPEAN PATENT SPECIFICATION

(45) Date of publication and mention of the grant of the patent:
**01.09.2004 Bulletin 2004/36**

(21) Application number: **98945748.6**

(22) Date of filing: **18.09.1998**

(51) Int Cl.$^7$: **C12N 15/63**, C12N 15/55, C12Q 1/37

(86) International application number:
**PCT/SG1998/000073**

(87) International publication number:
**WO 1999/015676 (01.04.1999 Gazette 1999/13)**

(54) **A NOVEL GENERATION OF CLONED HORSESHOE CRAB RECOMBINANT FACTOR C FOR DETECTION AND REMOVAL OF ENDOTOXIN**

EINE NEUE GENERATION DES REKOMBINANTEN FAKTOR C DER HUFEISENKRABBE ZUR ERKENNUNG UND ENTFERNUNG VON ENDOTOXIN

NOUVELLE GENERATION DE FACTEUR C DE RECOMBINAISON DE LIMULE CLONE DESTINE A LA DETECTION ET L'ELIMINATION D'ENDOTOXINE

(84) Designated Contracting States:
**DE FR GB IT**

(30) Priority: **19.09.1997 US 58816 P**
**21.05.1998 US 81767**

(43) Date of publication of application:
**05.07.2000 Bulletin 2000/27**

(73) Proprietor: **NATIONAL UNIVERSITY OF SINGAPORE**
**Singapore 119260 (SG)**

(72) Inventors:
- **DING, Jeak, Ling, Ind. Techn. Relations Office Singapore 119260 (SG)**
- **HO, Bow, Ind. & Techn. Relations Office Singapore 119260 (SG)**

(74) Representative: **Cripps, Joanna Elizabeth et al**
**Mewburn Ellis LLP**
**York House**
**23 Kingsway**
**London WC2B 6HP (GB)**

(56) References cited:
- **DATABASE WPI, Week 51, LONDON: DERWENT PUBLICATION LTD., AN 97-557571; & SG 42456 A1 (NATIONAL UNIVERSITY OF SINGAPORE) 15 August 1997.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### FIELD OF THE INVENTION

The present invention relates to recombinant Factor C (rFC) of a horseshoe crab, produced in an insect cell system. The invention also relates to vectors for producing the protein by recombinant DNA methods and to methods for using the recombinant Factor C to detect endotoxins in a sample or for removal of endotoxins from a sample by affinity methods.

### THE RELATED ART

The amoebocytes of horseshoe crabs contain an efficient coagulation cascade system which is activated by endotoxin, also known as lipopolysaccharide (LPS) from Gram negative bacteria. The enzymatic components of the coagulation cascade and the molecular events responsible for the subsequent gelation of the amoebocyte lysate have been characterized in *Tachypleus tridentatus*[1] and *Carcinoscorpius rotundicauda*[2,3,4]. Factor C has been shown to be the intracellular endotoxin-sensitive serine protease that initiates the coagulation cascade system[5].

By spiking, the LAL test detects femtogram levels of LPS[6]. Owing to its extreme sensitivity, the amoebocyte lysate, in particular, the *Limulus* amoebocyte lysate (LAL) has been developed into a commercial assay for widespread use in the detection of pyrogenic LPS in drugs and other pharmaceutical products[7,8]. This assay is based on the LPS-induced coagulation reaction of the lysate, culminating in formation of a gel clot. However, (a) the possible lack of specificity due to 1-3 β-D glucan and (b) the batch-to-batch variation in the sensitivity of commercial lysate to LPS, due to seasonal and geographical differences in the starting material[9] has prompted our laboratory to employ recombinant DNA technology to genetically-engineer Factor C as an alternative source of novel "limulus lysate" for endotoxin detection.

cDNAs encoding Factor C have been Cloned[1,10,15]. There are six potential glycosylation sites in the amino acid sequence of the Factor C from *Carcinoscorpius rotundicauda* (CrFC)[10,15]. Cloned cDNA encoding CrFC has been expressed in *E. coli*[11] and also in yeast expression systems[12,24]. The rFC obtained from yeast was found to be immunoreactive and capable of binding LPS, although only limited amounts of rFC produced in yeast were soluble[13,14]. Also, it was found that LPS could not activate the enzymatic activity of yeast rFC, thus, a direct enzyme-based LPS detection is not possible using rFC produced in yeast[14].

SG 42456 A discloses isolated *Carcinoscorpius rotundicauda* Factor C proteins with defined sequences of 1083 and 1019 amino acids. Further disclosed are an isolated and purified DMA encoding a protein having Factor C enzymatic activity useful in assays for Gram-negative bacterial endotoxin.

### SUMMARY OF THE INVENTION

The present inventors believed that expression in insect cells rather than in a prokaryotic or simple eukaryotic expression system is suitable for producing rFC with full biological activity. Furthermore, horseshoe crabs and insects belong to the same phylum, Arthropoda, and so insect cells might more closely resemble the cells of the horseshoe crab than yeast cells in their physiology and biochemistry. Thus, rFC produced in insect cells might more closely resemble the protein as purified from the horseshoe crab and retain the bioactivity of having a serine protease activity activated by LPS.

The present invention relates to genetic engineering of a bioactive rFC, which unequivocally exhibits full biological functionality. It is capable of specifically recognizing and binding LPS and Lipid A in both free and immobilized forms. Interference from 1-3 β-D-glucan, which switches on the alternate pathway in the coagulation cascade in conventional LAL, is not anticipated in assays of the present invention that use only Factor C as the LPS-binding, serine protease enzyme. Both the LPS-activated enzymatic assays of rFC and the ELISA lipid A binding assay could be formulated into a rapid high throughput mass screening test for LPS. Thus, a novel generation of "limulus amoebocyte lysate" has been invented, being capable of rapid and sensitive diagnosis and removal of subpiogram levels of endotoxin. The invention provides a standardized and convenient source of enzyme-based diagnostic reagent for detection of the ubiquitously contaminating endotoxin in pharmaceutical products. This inexhaustible supply of genetically-engineered Factor C can be easily standardized to circumvent batch-to-batch variations in sensitivity to LPS, a problem faced by the conventional LAL industry. Furthermore, the ability of the rFC of the invention to protect mice from endotoxemia, as well as its bacteriostatic activity, adds to its value in *in vivo* applications. Furthermore, the availability of rFC obviates the need for routine harvesting of the horseshoe crab for procurement of their amoebocyte lysate, and therefore, conserves this endangered "living fossil".

The present inventors have succeeded in expressing biologically active rFC using recombinant baculoviruses and insect host cells. The rFC obtained is enzymatically active. Thus, expression of rFC in insect cells is a convenient and economical source of rFC protein for use in rapid, sensitive, specific and quantitative determination of LPS in pharmaceutical products and other biological fluids.

Thus, the present invention comprises purified rFC that is enzymatically active. The phrase "enzymatically active" means that the Factor C protein has the biological activity of binding LPS or lipid A, being activated as to its serine protease activity upon LPS or lipid A binding. Enzymatically active rFC will induce coagulation of an amoebocyte lysate and will also cleave synthetic substrates such as, but not limited to, Boc-Val-Pro-Arg-MCA, Mu-Val-Pro-Arg-AFC and Boc-Val-Pro-Arg-pNA.

The present invention is also embodied in a method for producing substantially purified, enzymatically active rFC. The method comprises expressing DNA encoding a Factor C protein having the enzymatic activity described above in a culture of insect cells, then isolating the enzymatically active Factor C protein. The isolation preferably includes an ultrafiltration step. The purification preferably also includes a step of gel-filtration chromatography on a matrix having an exclusion limit of 100 kilodaltons. The gel filtration is preferably applied after the ultrafiltration. The exclusion limit of the gel filtration matrix can vary substantially; an effective matrix will provide at least about a 4-fold increase in the serine protease activity of an ultrafiltered crude preparation as measured by the fluorometric assay described herein.

The present invention also encompasses host-vector systems for expressing enzymatically active rFC. The host cells in these embodiments of the invention are insect cells, preferably leptidopteran cells. The vectors in these embodiments support replication of inserted DNA in insect cells and expression of heterologous DNA in insect cells. The vectors are preferably baculovirus or plasmid vectors. The heterologous DNA is sufficient to encode a Factor C enzyme of a horseshoe crab, preferably of the genus *Carcinoscorpius, Tachypleus* or *Limulus.* Preferred heterologous DNA is a polynucleotide having the sequence shown in SEQ ID NO: 1 or SEQ ID NO:3.

The present invention is also embodied in assays for endotoxin comprising contacting a sample to be assayed for the presence of endotoxin or LPS or Lipid A with enzymatically active rFC according to the invention and measuring the serine protease activity of the rFC. The amount of serine protease activity of the rFC will reflect its activation due to binding of LPS or Lipid A or of another endotoxin known in the art to bind to Factor C of a horseshoe crab. The serine protease activity is conveniently measured by any method known in the art but is preferably measured by a chromogenic or fluorogenic method. In such a method formation of a product from a substrate by cleavage of the substrate by the serine protease activity of the rFC, resulting in a change in color or in fluorescence emission, is measured. Preferred substrates for such a chromogenic or fluorogenic assay are N-t-BOC-Val-Pro-Arg-MCA, Mu-Val-Pro-Arg-AFC and Boc-Val-Pro-Arg-pNA.

Additional embodiments of the invention include immunologic methods for assaying the presence of Lipid A or LPS or endotoxin in a sample. These methods of the invention rely upon binding of antibody that specifically binds to Factor C and subsequent detection or quantitation of the amount of the Factor C-antibody complex. In a preferred embodiment, the sample to be assayed is contacted with immobilized antibody that specifically binds to Lipid A or LPS or endotoxin as the ligand to form immobilized ligand. The immobilized ligand is then contacted with rFC according to the present invention to form immobilized rFC. Then the immobilized rFC is contacted with a second antibody that specifically binds the rFC. Finally, the presence or preferably the amount of the rFC-second antibody complex is determined. This determination can be performed by any method typical in the art such as a third antibody that binds the second antibody, perhaps through its Fc portion, or the like. In an alternate embodiment of this aspect of the invention, the second antibody is omitted and the enzymatic activity of the immobilized rFC is measured.

In another embodiment of the invention, the specific binding of LPS or lipid A to rFC is employed in a BIACORE™ assay (Pharmacia Biotech). By immobilizing the rFC on the substrate plate of the BIACORE™ apparatus, the presence of LPS or lipid A in a sample can be detected. Optimization of the amount of the rFC to be immobilized for a given load of LPS in a sample is considered within the skill of the ordinary practitioner. The BIACORE™ apparatus is operated in accord with the manufacturer's instructions.

Also, the present invention is embodied in methods for removal of endotoxin from a sample, wherein immobilized rFC is contacted with the sample, under conditions such that endotoxin in the sample binds the immobilized rFC, then the bound endotoxin is separated from the sample.

According to a first aspect of the present invention there is provided a recombinant DNA vector comprising a baculovirus-derived vector and a cDNA encoding an enzymatically active Factor C of a horseshoe crab.

According to a second aspect of the present invention there is provided an assay for endotoxin comprising:

i) contacting a sample to be assayed, comprising a peptide cleavable to produce a chromogenic or fluorogenic moiety, with an enzymatically active recombinant Factor C of a horseshoe crab obtained by expression of a recombinant DNA vector in an insect host cell culture; and
ii) measuring the amount of said chromogenic or fluorogenic moiety cleaved from said peptide.

According to a third aspect of the present invention there is provided an assay for endotoxin comprising:

i) contacting a sample to assayed with an immobilized antibody that specifically binds to lipopolysaccharide or an immobilized antibody that specifically binds to lipid A to form a complex between said antibody and endotoxin in said sample;
ii) contacting said complex with an enzymatically active recombinant Factor C of a horseshoe crab obtained by expression of a recombinant DNA vector in an insect host cell culture to form immobilized Factor C; and
iii) contacting said immobilized Factor C with an antibody that specifically binds to said immobilized Factor C; and
iv) quantitating the amount of said antibody specifically bound to said immobilized Factor C.

According to a fourth aspect of the present invention there is provided a recombinant DNA vector comprising (i) a baculovirus-derived vector or a vector for expressing heterologous DNA in an insect cell line and (ii) a nucleic acid encoding a polypeptide having 75% or greater sequence identity to an amino acid sequence selected from the group consisting of SEQ. ID. NO. 2, SEQ. ID. NO. 4 and the amino acid sequence of Factor C of *Tachypleus tridentatus.*

According to a specific method of the present invention there is provided a method for removing endotoxin or lipid A from a sample comprising:

i) contacting immobilized recombinant Factor C of a horseshoe crab with said sample, so that endotoxin or lipid A in said sample binds to said immobilized recombinent Factor C; and
ii) separating said immobilized recombinant Factor C, having said endotoxin or lipid A bound thereto, from said sample,

wherein said recombinant Factor C is produced by expression of a recombinant DNA vector in an insect host cell culture.

BRIEF DESCRIPTION OF DRAWINGS

**Figure 1A-B: Cloning of CrFC21 cDNA into the baculovirus expression vector, pFastBac I™.** Two constructions of the same plasmid were done. (1A) A 2.3 kb HindIII fragment from pGEM11Zf(+)/CrFC21[15] was cloned into HindIII linearized pBluescript™ SK(+) (pBSSK) to give pBSSK/CrFC21/HH. This was then digested with Eco RI and ligated with the 2.3 kb Eco RI fragment from pGEM11Zf(+)/CrFC21 to regenerate recombinant pBSSK/CrFC21. Separately, a 1.3 kb XhoI/HindIII fragment, derived from pGEM11Zf(+)/CrFC21, was cloned into XhoI/HindIII digested pFastBacI™ to give pFastBac/CrFC21XH. The final full-length construct, pFastBac/CrFC21 was generated when the 2.9 kb Pst I CrFC fragment from pBSSK/CrFC21 was ligated to Pst I linearized pFastBac/CrFC21/XH.

(1B) The PfastBac I™ vector and an intermediate CrFC subclone of pGEM11Zf(+)/CrFCEN were digested with Eco RI and Hind III. The liberated CrFCEN insert was ligated directionally into the linearized pFastBac I™ vector to yield pFastBac/CrFCEN. pFastBac/CrFCEN was then digested with Eco RI, as was another vector comprising DNA encoding Factor C, pGEM11Zf(+)/CrFCEE. The insert released from pGEMIIZf(+)/CrFCEE was ligated into the linearized pFastBac/CrFCEN. The clone having the proper orientation was selected by restriction analysis and designated pFastBac/CrFC21'.

**Figures 2A to 2B: Immunoblot analysis of Sf9 rFC.** (2A) Reducing SDS PAGE of 5 µg total protein of cell lysate and culture supernatant harvested at 24 and 48 h post-induction (p.i.). (2B) Comparison of reducing and non-reducing SDS PAGE analyses of rFC from 72 h p.i. The results indicate that rFC is probably a double-chain form of Factor C as further proven by the LPS-treated (lane 2) and -untreated (lane 1) rFC under reducing and non-reducing conditions. Western Blots were developed using a horseradish peroxidase system.

**Figures 3A-3C: ELISA lipid A-binding assay of rFC.** (3A) A gradation of increasing intensity of color development of the enzymatically-hydrolyzed product is seen from 0.01 to 200 ng lipid A. Rows 1 & 2 contain 10 µg total protein per well of culture supernatant from pFastBac/CrFC21 infected Sf9 cells after 72 h p.i. Rows 3 & 4 are controls containing 10 µg total protein per well of culture supernatant from wild-type ACMNPV-infected Sf9 cells after 72 h p.i. (3B) The histogram illustrates a quantification of the lipid A based on the absorbance at $OD_{405nm}$ of 72 h culture supernatant (10 µg) and cell lysate (20 µg) after their reaction with 0.01 to 200 ng of lipid A. The results were normalized with wild type baculovirus infected samples. The culture supernatant consistently showed higher efficacy of binding lipid A even though a lower total amount of protein (10 µg) was used. (3C) Blocking of excess sites with 0.2% BSA effectively removed the non-specific background binding, and results in higher net absorbance readings.

**Figure 4 : Biological Activity of BIOMAX™ purified rFC**. The rFC was enriched by BIOMAX™-50 ultrafiltration and this protein sample was reacted with a range of absolute amounts of LPS (0.01-10 000 pg). Both 40 and 80 µg amounts of rFC showed enzymatic activity with 0.01 pg LPS.

**Figure 5: Fluorimetric and colorimetric assays of LPS using purified rFC.** In both the fluorimetric and colorimetric assays, further purification of BIOMAX™-purified rFC by SEPHADEX™ G-100 dramatically improved the sensitivity of rFC to LPS. The conventional tube method of fluorimetric assay was compared with the microcolorimetric assay for both the BIOMAX™ sample (B) and SEPHADEX™ G-100 purified sample (S). Amounts of rFC used were 40 µg and 100 µg for the fluorimetric and colorimetric assays, respectively.

**Figure 6: Comparison of tube and plate fluorimetric assays.** A comparison of the fluorescence readings was made between the conventional tube method and the microtiter plate method using 40 µg of the SEPHADEX™ G-100 purified rFC.

**Figure 7: Colorimetric assay for LPS-induced Factor C enzyme activity of rFC**. A comparison is made of the amounts of culture supernatant proteins (60 and 100 µg) containing rFC, and the concentrations (2 and 4 mM) of the colorimetric substrate, Boc-Val-Pro-Arg-pNA. It is observed that as low as 0.01 pg endotoxin was optimally detected with 100 µg culture supernatant at 2 mM pNA substrate.

**Figure 8: Colorimetric assay of LPS using purified rFC.** Similar to the fluorimetric assay, the colorimetric test also showed that the SEPHADEX™ G-100 purified rFC exhibited improved sensitivity to LPS, where 40 µg of purified rFC (instead of 100 µg of BIOMAX™-purified rFC) was sufficient to detect subpicogram levels of LPS.

**Figure 9: Binding of rFC to lipid A assayed by BIACORE™ bioassay.** Binding between rFC produced from pFastBac/CrFC21 and immobilized lipid A (*E. coli* D31m4) was assayed using the BIACORE™ X biosensor (Pharmacia Biotech). BIOMAX™ and SEPHADEX™ G-100 purified supernatants of cultures of Sf9 cells infected with wild-type baculovirus did not show any background binding to the immobilized lipid A (plateau 1A). On the other hand, the rFC from the culture supernatant of Sf9 cells infected with pFastBac/CrFC21 specifically bound the immobilized lipid A with a net activity of 553 Response Units (plateau 2A). The protein samples, each at 1 mg/ml, were injected at 10 µl/min for 3 minutes over the ligand monolayer that was previously immobilized on a HPA chip[14].

**Figure 10: rFC from Baculovirus binds LPS and lipid A from several Gram negative bacterial species.** LPS from three different species of bacteria, *E coli, K. pneumoniae*, and *S. minnesota,* and lipid A from *S. minnesota,* were separated by electrophoresis and electroblotted onto an Immobilon™ PVDF membrane. Each LPS/lipid A strip was incubated with rFC from pFastBac/CrFC21 (lane 1) or control culture supernatant from wild-type AcMNPV-infected Sf9 cells (lane 2). The results show that rFC binds LPS/lipid A from different species of Gram negative bacteria.

**Figure 11: Microtiter plate-immobilized rFC for detection and removal of LPS**. 10, 25 or 50 µg of partially-purified protein containing rFC derived from baculoviral system (rFC Sf9) immobilized on a 96-well microtiter plate was capable of specifically recognizing and binding subprogram levels of FITC-conjugated LPS. The efficacy of binding/detection of a range of LPS by various amounts of rFC protein immobilized onto the microtiter plate is shown.

**Figure 12: Microtiter plate-immobilized rFC from various yeasts binds to LPS.** Immobilized rFC derived from yeast (*P. pastoris:* rFC#8 {pHILD2/CrFC21} and rFCEE {pHILD2/CrFC21EE}; *S. cerevisiae:* YFC/6a {YepSec1/CrFC26Δ6a} and P21/26 {pEMBLyex4/CrFC21/26}). Native Factor C in *Carcinoscorpius* amoebocyte lysate, LAL (50 µg protein) were used as positive controls. There is consistency in the efficiency of recognition of LPS-FITC and its binding to the immobilized rFC. The negative controls were w/tSf9 (wild-type Sf9 cells infected with AcMNPV DNA alone) and rFCSN (rFC derived from a control yeast recombinant clone devoid of the LPS binding domain).

## DETAILED DESCRIPTION OF THE INVENTION

The present application relates to rFC of the horseshoe crab.

A preferred horseshoe crab that can serve as a source of DNA or mRNA for producing the rFC of the invention is *Carcinoscorpius rotundicauda* (CrFC). The present invention relates especially to expression of rFC by means of baculovirus host-vector systems. The present application also relates to a fluorometric assay for endotoxin that makes use of the rFC expressed by recombinant DNA methods.

cDNAs encoding Factor C proteins from *Carcinoscorpius rotundicauda* have been previously described[10,15]. rFC from *Carcinoscorpius rotundicauda* (rCrFC) has been produced *in vitro* by coupled transcription/translation systems[10,15]. However, the present invention resides partly in the development of *in vivo* systems, especially using insect cells as the host cell, for efficient production of rFC by expression of cloned DNA.

Also, the protection of rFC from activation and subsequent self-proteolysis by binding of endotoxin which may be present in solutions used in isolation of the protein is described in reference 15. Basically, dimethylsulfoxide ($Me_2SO$, DMSO) is added to solutions which are used during the purification process. Even greater protection of the rFactor C is achieved by also adding an agent effective for chelating divalent metal ions to the purification solutions.

cDNAs appropriate for expression in the presently-described system can be cDNAs encoding Factor C of any horseshoe crab. Two representative nucleotide sequences are presented as SEQ ID NO:1 and SEQ ID NO:3 (encoding the amino acid sequences of SEQ ID NOs:2 and 4). A composite DNA sequence, assembled from incomplete cDNA fragments, encoding the Factor C of *Tachypleus tridentatus* is disclosed by Muta et al.[1]

For use in the LPS binding assays and LPS removal according to the invention, the Factor C can be produced by any method typical in the art, but is preferably made in a eukaryotic host cell. Production of rFC in yeast host-vector systems is described in reference 16. As it has been the Inventors' recent experience that Factor C produced in yeast lacked serine protease activity, rFC for use in enzymatic activity-based assays is preferably produced by a baculovirus host-vector system.

"Stringent conditions" for hybridization are those that provide for hybridization of sequences having less than 15% mismatch, preferably less than 10% mismatch, most preferably 0% to 5% mismatch. Exemplary of such conditions, using probes of 50 bases or longer, are an aqueous solution of 0.9 M NaCl at 65 °C; an aqueous solution of 0.98 M NaCl, 20% formamide at 42-45 °C. The conditions will vary according to the length of the probe, its G+C content and other variables as known to the skilled practitioner[17]. Exemplary wash conditions following hybridization are an aqueous solution of 0.9 M NaCl at 45-65 °C, preferably 55-65 °C. Lower salt, or addition of an organic solvent such as formamide, in the wash buffer will increase the stringency of the condition as known in the art.

A preferred hybridization condition is at 42°C in 50% formamide, 5x SSC, 1x Denhardt's solution, 20 mM phosphate buffer, pH 6.5, 50 µg/ml calf thymus DNA, 0.1% SDS. Salt and temperature conditions equivalent to the hybridization conditions employed can be calculated from the following equation[18]:

$$T_m = 81.5^\circ C - 16.6(\log_{10}[Na^+]) + 0.41(\%G{+}C) -$$

$$0.63(\%formamide) - (600/l),$$

where $l$ = the length of the hybrid in base pairs.

A preferred washing condition is in 1x SSC, 0.1% SDS washing solution at room temperature, followed by washing at high stringency with 0.1x SSC, 0.1% SDS at 42°C and 2x with 0.1x SSC/0.1% SDS for 15 min. each at 42°C.

**Example 1: Recombinant constructs of CrFC cDNA in a baculovirus expression vector**

*Plasmids and Sf9 cell culture*

Sf9 insect cells were maintained as a monolayer culture in serum-free SF 900 II SFM medium supplemented with 50 U/ml penicillin and 50 µg/ml streptomycin (Life Technologies, Inc.) in a humidified incubator (Forma, USA) at 27°C. The plasmid pFastBac I™ and the competent DH10Bac *E. coli* were from Life Technologies, Inc., USA.

*Construction of pFastBac/CrFC21, transposition into E. coli and transfection into Sf9 insect cells.*

The strategy for cloning CrFC21 into the pFastBac I™ (Life Technologies, Inc.) expression shuttle vector is shown in Fig 1. The recombinant plasmids were verified by restriction enzyme digestion. The 5' cloning sites were further confirmed by dideoxynucleotide sequencing using the forward primer designed from the -44 position of the polyhedrin promoter region, before they were used for transfection in insect cells. PCR and Southern analyses of the pFastBac/CrFC21 DNA confirmed the authenticity of recombinant baculoviruses.

The CrFC21 cDNA[10] from pGEM11Zf+/CrFC21[11] was recloned in two steps into pBluescript II SK+ (pBSSK), to yield pBSSK/CrFC 21. Further manipulations using pBBSK/CrFC21 and the baculoviral expression vector, pFastBac I™ were carried out using standard methods to clone full-length CrFC21, thus, yielding the recombinant construct, pFastBac/CrFC21 (Fig. 1). pFastBac/CrFC21 was transformed into competent *E. coli,* DH10Bac, and cultured in LB agar containing 50 µg/ml kanamycin, 7 µg/ml gentamycin, 10 µg/ml tetracycline, 30 µl of 2% X-gal and 40 µg/ml of IPTG. Screening[19] for positive clones involved the use of the 2.3 kb $^{32}$P-CrFC21/EE fragment as probe[10]. The recombinant bacmid DNA was isolated and transfected into Sf9 cells.

**Example 2: Expression of rFC in insect host cells**

*Rapid microtiter-plate plaque assay*

Early log phase recombinant Sf9 cells were seeded at 6.5 x $10^4$ cells per well. The culture was incubated in a sealed bag at 27 °C for 1 h. Meanwhile, the virus stock was serially diluted 10-fold with SFM containing 10% FBS to give final dilutions of $10^{-2}$ to $10^{-4}$. The BacPak™ Baculovirus Rapid Titer Kit (InVitrogen) was used for plaque assay. It is an immunoassay which uses a primary monoclonal antibody raised to an AcMNPV envelope glycoprotein (gp64).

A secondary goat anti-mouse HRP-conjugated antibody enables visualization of the infected cells as blue-stained viral plaques or foci seen under the light microscope. The virus titer (pfu/ml) was calculated based on the following formula : (Average no. of foci per well x dilution factor x 40) x 2 where 40 represents the inoculum volume normalization factor.

*Scale-up of infection of Sf9 cells for production of rFC*

The culture supernatant from the 6-well plates was harvested and the viral stock was amplified by re-infection of Sf9 cells grown in 25 $cm^2$ flasks, using a multiplicity of infection (MOI) of 0.1 - 1.0. In such cultures, the viral stock reached a titer of 2 x $10^7$ pfu/ml. Aliquots of this viral stock were re-inoculated at a MOI of 5-10 into Sf9 cells grown in 15 ml SFM medium in 75 $cm^2$ flasks. The volume of the viral inoculum was determined using the formula:

$$\frac{\text{(total no. of cells) x (MOI in pfu/cell)}}{\text{(viral titer in pfu/ml)}}$$

Subsequently, Sf9 cells were passaged twice and conditioned to grow in suspension in 100 ml SFM medium, in spinner flasks (Bellco, USA). At the mid log phase of growth, the viral stock from the 75 $cm^2$ flask cells was inoculated at a MOI of 5-10. In the same manner, the cell culture volume was scaled up further in increasingly larger spinner flasks of 250, 500 and 1000 ml, infected with proportionally increasing volumes of viral stock at the same MOI.

*Preparation ofprotein samples from recombinant baculovirus-infected Sf9 cells.*

(a) Cell lysate: Sf9 cells infected with the recombinant baculovirus at a MOI of 5-10 were harvested at 24, 48 and 72 h p.i. The cells were washed 3 times with pyrogen-free PBS and centrifuged at 3000 xg for 10 min at 4°C during each cycle of washing. The cell pellet was resuspended in 2-3 volumes of PBS and subjected to 5 cycles of freeze-thawing at -80°C and 37°C, respectively. The cell debris was removed by centrifuging at 14000 xg for 10 min at 4°C. The supernatant containing the soluble protein fraction was stored at -20°C. This supernatant represents the cell lysate.

(b) Culture supernatant: At the respective times of harvest, the cell medium was collected and centrifuged at 3000 xg for 10 min at 4°C to remove any cells or cell debris. The medium was then concentrated 10-fold by centrifugation through a BIOMAX™-50 kDa cutoff ultrafree membrane (Millipore) at 2000 xg for 20 min or more. The total proteins present in the cell lysate and culture supernatant were quantified by Bradford assay[20]. Partial purification of rFC was carried out at 4 °C by gel filtration chromatography through SEPHADEX™ G-100 (*e.g.* 1.5 x 90 cm), using 0.05 M Tris-HCl (pH 7.5) containing 0.154 M NaCl. Fractions of 1 ml were collected and the void volume peak was concentrated. The protein concentration and Factor C enzyme activity were assayed for the resulting rFC. This preparation is henceforth referred to as "gel filtration-purified rFC".

*Western immunoblot detection of rFC*

Five µg of each cell lysate, or culture supernatant, harvested from 24, 48 and 72 h p.i. was analyzed on 10% SDS-PAGE gels, under denaturing conditions[21]. The electrophoretically-resolved bands were then transferred onto Immobilon™ PVDF membrane (Millipore, USA). The membrane was washed in PBS for 30 min, and blocked in 1% skimmed milk-PBS for 1 h followed by overnight incubation with rabbit anti-Factor C antibody diluted 1:500 in 0.2% Tween-20-PBS containing 1% BSA. Horseradish peroxidase-conjugated secondary goat anti-rabbit antibody, diluted 1:10000, was subsequently incubated with the membrane. For visualization of protein bands, the membrane was treated with SUPERSIGNAL™ chemiluminescent substrate (Pierce, USA) for 5-10 min, followed by 3 min exposure of the membrane to an X-ray film.

The Western analysis revealed 3 bands of immunoreactive rFC proteins of 132, 88 and 44 kDa, expressed by pFastBac/CrFC21 recombinant baculoviruses at 24, 48 and 72 h post infection, p.i. (Fig. 2A). At 24 h p.i., rFC was observed in the culture supernatant, but not in the cell lysate. The 48 h and 72 h p.i. culture supernatant showed increasing amounts of rFC. The rFC in the cell lysate started to appear as a faint 132 kDa band only at 48 h p.i., and reached a substantial level at 72 h p.i. (Fig. 2B). The immunoblot thus showed that the bulk of the rFC produced was released from the infected Sf9 cells into the medium. This is probably due to lysis of the infected cells, which released the recombinant protein. On ultracentrifugation at 100,000 xg for 1 h at 4 °C, rFC was found to be in the soluble fraction.

The results show that rFC protein was expressed correctly under the direction of the viral late promoter from the polyhedrin gene using the native translation start site from the CrFC cDNA. As there are six potential glycosylation sites in the CrFC cDNA sequence[10]; the protein band of 132 kDa represents the intact glycosylated form of Factor C.

The 88 and 44 kDa proteins are likely the activated products of rFC whose molecular sizes correspond closely to the heavy and light chains, respectively, of double-chain Factor C[3]. Autoactivation could have occurred in the presence of picogram levels of ubiquitous endotoxin during the preparation of the protein sample for SDS-PAGE. Figure 2B shows a comparison of rFC from 72 h p.i., electrophoresed under reducing and non-reducing conditions of SDS-PAGE. The 88 and 44 kDa bands became more prominent under reducing conditions. Under non-reducing conditions, LPS-activated rFC still retained its 132 kDa band, thus indicating the double-chain form of rFC[3]. The presence of a double chain form of rFC was further proven when the BIOMAX™-purified rFC was pre-incubated with LPS before Western blotting. Under reducing condition, the LPS-treated rFC showed activated products of 88 and 44 kDa which were absent in the untreated rFC sample (Fig. 2C). However, under non-reducing conditions (Fig. 2D), the 132 kDa band was intact for both the LPS-treated and -untreated rFC.

**Example 3: rFC binds lipid A, the biologically-potent component of LPS**

*ELISA to determine lipid A binding by rFC*

In order to visualize and test the ability of rFC to specifically bind the biologically potent component of LPS, diphosphoryl lipid A (*E. coli* K12, D31M4, List Biologicals, Inc., USA) ranging from 0.01 to 100 ng in 100 µl volumes was immobilized onto 96-well Nunc IMMUNO-PLATES™ (PolySorp). The immobilization was carried out overnight at room temperature. Unbound lipid A was removed and the plates were washed 6 times with wash buffer containing 0.01% Tween 20 and 0.01% thimerosal in PBS. The excess sites were blocked for 1 h at room temperature with the same buffer containing 0.2% BSA, after which the wells were again washed 6 times. Aliquots of 100 µl of BIOMAX™ 50-treated rFC from culture supernatant containing 20 µg total protein was then added to the wells and incubated overnight at room temperature. Unbound rFC was removed, and the wells were washed 6 times in wash buffer. This was followed by addition of aliquots of 100 µl of 1:500 diluted rabbit anti-Factor C antibody and incubation was continued for 2 h at 37°C. Subsequently, the wells were washed 6 times in the wash buffer before addition of 100 µl aliquots of 1:2000 diluted goat anti-rabbit antibody conjugated with horseradish peroxidase. After washing the wells 6 times, 1 mg/ml of substrate ABTS, 2,2'-azino-di-[3-ethylbenzthiazoline sulfonate (6)] diammonium salt (Boehringer Mannheim) was added in 200 µl aliquots and incubated at room temperature for 15 min. The formation of a green product was quantified by reading its absorbance at 405 nm. rFC binding the immobilized lipid A results in positive color formation via the ELISA test.

The ELISA test for lipid A-binding indicates that rFC is capable of specifically recognizing and binding to immobilized lipid A (Fig. 3) and hence, it could be used in the detection of endotoxin. With increasing amounts of lipid A in the wells, there is an increasing intensity of color development in the ABTS product (Fig. 3A). Compared to the cell lysate, the 72 h p.i. culture supernatant consistently yielded more efficacious rFC for detection of lipid A (Fig. 3B). Furthermore, it was observed that blocking of excess sites in the wells with 0.2% BSA removed non-specific background binding and drastically improved the specificity of lipid A binding (Fig. 3C). This assay indicates that rFC can be used for mass screening of pharmaceutical products for LPS contamination, with the capability of quantifying LPS. This efficacy is comparable to the commercially available natural lysate derived from the *Limulus* or *Tachypleus* amoebocyte lysate.

**Example 4: Immobilized rFC can be used to detect/remove LPS in a sample:**

Two hundred µl samples containing either control wild-type supernatant (w/t Sf9, uninfected Sf9 cell supernatant) or partially-purified rFC samples (obtained by BIOMAX™-ultrafiltration), diluted in PBS to 10, 25 or 50 µg total protein per 200 µl were coated/immobilized onto each of the wells of a 96-well microtiter plate (NUNC, USA). The plates were left overnight at 4°C. Unbound protein was removed from the wells, and 200 µl of 0.2% BSA (depyrogenized by ultrafiltration) dissolved in PBS was added to the wells for 1 h at 37°C, to block unoccupied sites. The wells were washed 3 times with PBS. This was followed by addition of 200 µl FITC-conjugated LPS (*E. coli* 055:5B, List Biological Labs, USA) to the wells. The plate was incubated at 37°C for 1 h, after which each well was washed 6x with PBS. The fluorescence was read at $Ex_{495nm}$ and $Em_{525um}$ using LS-50B Spectrofluorimeter (Perkin Elmer).

Wells coated with 10, 25 or 50 µg of partially purified proteins containing rFC showed increasing efficiency of binding LPS-FITC. Blocking of the wells with 0.2% BSA reduced the background fluorescence reading, indicating improvement in the specificity of binding of LPS to the immobilized rFC. Immobilization of negative control proteins (w/tSf9: wild-type Sf9 cell culture supernatant of Sf9 cells infected with AcNMPV DNA alone, and rFCSN: yeast rFC derived from the truncated recombinant rFC devoid of LPS-binding domain described in ref. 16, to the wells did not capture or bind LPS, thus indicating the specificity of recognition of LPS by the immobilized rFC.

**Example 5: The baculoviral rFC is enzymatically activated by LPS**

*Fluorimetric and colorimetric assays for LPS-activated rFC enzyme activity*

As a proenzyme, Factor C becomes catalytically activated by trace levels of LPS. Thus, conversion of its enzymatic substrate to product indicates the presence of LPS. rFC samples present in the crude cell lysate and culture supernatant were used for analysis of LPS-activated Factor C enzyme activity by using two different substrates. The first, in a conventional tube assay format, is based on a modification of the fluorimetric assay of Iwanaga *et al*.[22] Using rFC obtained from a 72 h p.i. culture supernatant, 10 μg total protein in a volume of 0.1 ml was mixed with 1.9 ml of 50 mM Tris-HCl, pH 8.0, containing 0.1 M NaCl and 0.05 M $CaCl_2$. The mixtures were preincubated with 0.01 to 100 pg of LPS *(E. coli* 055:B5, Sigma) at 37 °C for 1 h before addition of 15 μl of 2 mM fluorimetric substrate, Boc-Val-Pro-Arg-MCA (Sigma). Incubation was continued for 30 min and the reaction was terminated with 0.1 ml glacial acetic acid. The product AMC was read in Fluorescence Units (FU) at $Ex_{380nm}$ (slit 10 nm) and $Em_{460nm}$ (slit 5 nm) using a Perkin Elmer Luminescence Spectrophotometer (LS-50B). For multiple samples, this assay was routinely scaled down to 96-well microtiter plate assay. Briefly, the microassay involved 1 h preincubation of LPS with rFC in a volume of 100 μl, followed by addition of 1.5 μl of 2 mM fluorimetric substrate and 100 μl of 100 mM Tris-HCl, pH 8.0, containing 0.2 M NaCl and 0.1 M $CaCl_2$ and further incubation for 30 min at 37 °C before termination of the reaction with 10 μl of glacial acetic acid. The fluorescence was read in a 96-well microtiter plate reader module.

The second enzymatic assay for LPS involved a modification of the colorimetric test[23] where preincubation of culture supernatant proteins with LPS ranging from 0.01 to 10 pg was carried out at 37 °C for 1 h. The reaction volume was scaled down to 200 μl in 0.1 M Tris-HCl (pH 8.0) containing 5 mM $MgCl_2$. This was followed by addition of 50 μl of 2 mM of a colorimetric substrate, Boc-Val-Pro-Arg-p-nitroanilide (Seikagaku, Japan). Incubation at 37 °C was resumed for 1 h before termination of the reaction with 28 μl of glacial acetic acid. This substrate is hydrolyzed by rFC to produce pNA that was measured colorimetrically at $OD_{405nm}$.

From 24 to 48 to 72 h p.i., there was progressively increasing trend in the enzymatic activity of rFC in supernatants of cultures of insect cells transformed with the construct of Example 1, as indicated by the increase in fluorimetric units of the AMC product hydrolyzed from Boc-Val-Pro-Arg-MCA substrate. A comparison of the amount of total proteins present in the cell lysate (Lysate: 50 μg) and culture supernatant (Sup: 5 μg) illustrates that the culture supernatant from 72 h p.i. contained rFC that is >5-10 fold more effective in LPS detection. Twenty μg of BIOMAX™-purified rFC was able to detect 0.01 ng LPS. Using 40 to 80 μg of this protein, the detection limit could be easily extended to LPS levels below 0.01 pg or 0.001 ng/ml (Fig. 4). Purification of rFC by chromatography through SEPHADEX™ G-100 yielded enzymatic activity of even higher sensitivity to LPS (Fig. 5). It is envisaged that more elaborate purification of rFC following the methods covered in reference 24 would vastly improve the efficacy of the rFC for endotoxin detection. Furthermore, when the fluorimetric assay was modified to ~ 200 μl, using a 96-well microtiter plate, the sensitivity to LPS was improved by 10-fold (Fig. 6). This was directly attributable to the removal of background fluorescence by gel filtration.

Furthermore, the LPS-activated rFC enzyme assay was also conveniently quantifiable by a colorimetric assay with the Boc-Val-Pro-Arg-pNA substrate. The sensitivity to LPS was 0.1 pg (0.01 ng/ml) with 100 μg of BIOMAX™-50-treated culture supernatant when 2 mM of the pNA substrate was employed (Fig. 7). Similar to the fluorimetric assay, the colorimetric test also showed that the SEPHADEX™ G-100-purified rFC exhibited improved sensitivity to LPS, where 40 μg of purified rFC (instead of 100 μg of BIOMAX™ rFC) was sufficient to detect subpicogram levels of LPS (Fig. 8). Use of gel filtration-purified rFC resulted in a 4-fold increase in sensitivity to LPS. A direct comparison of the 2 microassays revealed that with gel filtration-purified rFC, the colorimetric assay achieved sensitivity to LPS comparable to the fluorimetric assay. Thus, using the scaled down, yet improved sensitivity assay for LPS detection, high throughput screening of samples can be conveniently achieved by either the colorimetric or fluorimetric assay using the 96-well microtiter plate assays. This enables rapid and mass screening of samples with limited volumes.

The invention being thus described, modification of the invention with respect to various materials and methods will be apparent to one of ordinary skill in the art. Such modifications are to be considered as falling within the scope of the invention, which is defined by the claims hereinbelow.

**References**

Articles of the scientific and patent literature referred to herein are incorporated by reference in their entirety by citation thereto.

1 Muta, T., Miyata, T., Misumi, Y., Tokunaga, F., Nakamura, J., Toh, Y., Ikehara, Y., and Iwanaga, S. 1991. *J Biol. Chem.* **266**: 6554-6561.

2 Navas, M.M.A, Ding, J.L., and Ho, B. 1990. Inactivation of Factor C by dimethyl sulphoxide inhibits coagulation of the *Carcinoscorpius* amoebocyte lysate. *Biochem Mol Biol Int* **21**:805-813.

3 Ding, J.L., Navas, M.M.A., and Ho, B. 1993. Two forms of Factor C from the amoebocytes of *Carcinoscorpius rotundicauda*: purification and characterization. *Biochim Biophys Acta* **1202**:149-156.

4 Ho, B., Kim, J.C., and Ding, J.L. 1993. Electrophoretic analysis of endotoxin-activated gelation reaction of *Carcinoscorpius rotundicauda* amoebocyte lysate. *Biochem Mol Biol Int* **29**:687-694.

5 Iwanaga, S. 1993. The limulus clotting reaction. *Current Opinion in Immunol* **5**:74-82.

6 Ho, B. 1983. An improved *Limulus* gelation assay. *Microbios Lett* **24**:81-84.

7 Cooper, J.F. 1975. Principles and applications of the limulus test for pyrogen in parenteral drugs. *Bull. Parent. Drug Ass.* **29**: 122.

8 Novitsky, T. J. 1991. Discovery to commercialization: the blood of the horseshoe crab. *Oceanus* **27**: 13-18.

9 Sekiguchi, K. and Nakamura, K. 1979. Ecology of the extant horseshoe crabs. In: Biomedical Applications of the Horseshoe Crabs (*Limulidae*), Eds., Cohen *et al*., Allan R. Liss, New York, pp. 37-49.

10 Ding, J.L., Navas III, M.A.A., and Ho, B. 1995. Molecular cloning and sequence analysis of Factor C cDNA from the Singapore horseshoe crab, *Carcinoscorpius rotundicauda. Mol Marine Biol Biotechnol* **4**:90-103.

11 Roopashree, S.D., Chai, C., Ho, B, and Ding, J.L. 1995. Expression of *Carcinoscorpius rotundicauda* Factor C cDNA. *Biochem Mol Biol Intl* **4**:841-849.

12 Roopashree, S.D., Ho, B, and Ding, J.L. 1996. Expression of *Carcinoscorpius rotundicauda* Factor C in *Pichia pastoris. Mol Marine Biol Biotechnol* **5**: 334-343.

13 Ding, J.L., Chai, C., Pui, A.W.M. and Ho, B. 1997. Expression of full length and deletion homologues of *Carcinoscorpius rotundicauda* Factor C in Saccharomyces cerevisiae: immunoreactivity and endotoxin binding. *J Endotoxin Res.* **4**(1): 33-43.

14 Pui, A.W.M., Ho, B. and Ding, J.L. 1998. Yeast recombinant Factor C from horseshoe crab binds endotoxin and causes bacteriostasis. *J. Endotoxin Research* (in press).

15 U.S. Patent 5,716,834.

16 Copending U.S. patent application Serial No. 08/596,405.

17 Sambrook et al., Molecular Cloning: A Laboratory Manual, $2^{nd}$. ed., c. 1989 by Cold Spring Harbor Laboratory, Cold Spring Harbor, NY.

18 *ibid*. pp. 9.50-9.51.

19 Grunstein, M., and Hogness, D.S. 1975. Colony hybridization: A method for the isolation of cloned DNAs that contain a specific gene. *Proc Natl Acad Sci* **72**:3961.

20 Bradford, M.M. 1976. A rapid and sensitive method for the quantitation of microgram quantities of protein utilizing the principle of protein dye binding. *Anal Biochem* 72:248-254.

21 Laemmli, U.K. 1970. Cleavage of structural proteins during the assembly of the head of bacteriophage T4. *Nature* **227**:680-685.

22 Iwanaga, S., Morita, T., Ohki, M. 1980. Endotoxin-sensitive substance. Japan Patent Agency Official Bulletin; S57-108018.

23 Nakamura, T., Morita, T., and Iwanaga, S. 1986 Lipopolysaccharide-sensitive serine protease zymogen (Factor C) found in Limulus hemocytes: Isolation and characterization. *Eur J Biochem* **154**: 511-521.

24 U.S. Patent 5,712,144.

## Claims

1. A recombinant DNA vector comprising a baculovirus-derived vector and a cDNA encoding an enzymatically active Factor C of a horseshoe crab.

2. The vector of claim 1, wherein said baculovirus-derived vector is pFastBacI.

3. The vector of claim 2, that is pFastBac/CrFC21.

4. The vector of claim 1, wherein said cDNA encoding an enzymatically active Factor C comprises a polynucleotide that hybridizes to a nucleotide sequence selected from SEQ ID NO:1, the complement of SEQ ID NO:1, SEQ ID NO:3, the complement of SEQ ID NO:3 or a cDNA from *Tachypleus tridentatus* encoding a Factor C enzyme, under stringent conditions.

5. The vector of claim 1, wherein said cDNA encoding an enzymatically active Factor C comprises a polynucleotide that encodes an amino acid sequence selected from SEQ ID NO:2, SEQ ID NO:4, or the amino acid sequence of Factor C from *Tachypleus tridentatus*.

6. The vector of claim 1, wherein said cDNA encoding an enzymatically active Factor C comprises a polynucleotide having the sequence of SEQ.ID.NO.1 or SEQ ID NO:3.

7. An assay for endotoxin comprising:

   i) contacting a sample to be assayed, comprising a peptide cleavable to produce a chromogenic or fluorogenic moiety, with an enzymatically active recombinant Factor C of a horseshoe crab obtained by expression of a recombinant DNA vector according to any of claims 1 to 6 in an insect host cell culture; and
   ii) measuring the amount of said chromogenic or fluorogenic moiety cleaved from said peptide.

8. The assay of claim 7, wherein said peptide is N-t-Boc-Val-Pro-Arg-MCA, Mu-Val-Pro-Arg-AFC or Boc-Val-Pro-Arg-pNA.

9. The assay of claim 7 or 8, wherein said insect host cell is a lepidopteran cell.

10. An assay for endotoxin comprising:

   (i) contacting a sample to be assayed with an immobilized antibody that specifically binds to lipopolysaccharide or an immobilized antibody that specifically binds to lipid A to form a complex between said antibody and endotoxin in said sample;
   ii) contacting said complex with an enzymatically active recombinant Factor C of a horseshoe crab obtainable by expression of a recombinant DNA vector according to any of claims 1 to 6 in an insect host cell culture; and
   iii) contacting said immobilized Factor C with an antibody that specifically binds to said immobilized Factor C; and
   iv) quantitating the amount of said antibody specifically bound to said immobilized Factor C.

11. The assay of claim 10, wherein said insect host cell is a lepidopteran cell.

12. A method for removing endotoxin or lipid A from a sample comprising:

   i) contacting immobilized recombinant Factor C of a horseshoe crab with said sample, so that endotoxin or lipid A in said sample binds to said immobilized recombinant Factor C; and

ii) separating said immobilized recombinant Factor C, having said endotoxin or lipid A bound thereto, from said sample;

wherein said recombinant Factor C is produced by expression of a recombinant DNA vector according to any of claims 1 to 6 in an insect host cell culture.

**13.** A recombinant DNA vector comprising(i) a baculovirus-derived vector or a vector for expressing heterologous DNA in an insect cell line and (ii) a nucleic acid encoding a polypeptide having 75% or greater sequence identity to an amino acid sequence selected from the group consisting of SEQ. ID. NO. 2, SEQ. ID. NO. 4 and the amino acid sequence of Factor C of *Tachypleus tridentatus*, wherein the recombinant DNA vector leads to the expression of enzymatically active Factor C of a horseshoe crab.

**14.** The recombinant DNA vector of claim 13, wherein said nucleic acid (ii) is obtained from a horseshoe crab of the genus *Limulus*.

**Patentansprüche**

**1.** Rekombinanter DNA-Vektor, umfassend einen von Baculovirus abgeleiteten Vektor und eine cDNA, die für einen enzymatisch aktiven Faktor C eines Molukkenkrebses kodiert.

**2.** Vektor nach Anspruch 1, worin der von Baculovirus abgeleitete Vektor pFast-Bacl ist.

**3.** Vektor nach Anspruch 2, der pFastBac/CrFC21 ist.

**4.** Vektor nach Anspruch 1, worin die für einen enzymatisch aktiven Faktor C kodierende cDNA ein Polynucleotid umfasst, das unter strengen Bedingungen an eine Nucleotidsequenz hybridisiert, die aus Seq.-ID-Nr. 1, dem Komplement von Seq.-ID-Nr. 1, Seq.-ID-Nr. 3, dem Komplement von Seq.-ID-Nr. 3 oder einer cDNA von Tachypleus tridentatus, die für ein Faktor-C-Enzym kodiert, ausgewählt ist.

**5.** Vektor nach Anspruch 1, worin die für einen enzymatisch aktiven Faktor C kodierende cDNA ein Polynucleotid umfasst, das für eine Aminosäuresequenz kodiert, die aus Seq.-ID-Nr. 2, Seq.-ID-Nr. 4 oder der Aminosäuresequenz von Faktor C von Tachypleus tridentatus ausgewählt ist.

**6.** Vektor nach Anspruch 1, worin die für einen enzymatisch aktiven Faktor C kodierende cDNA ein Polynucleotid umfasst, das die Sequenz von Seq.-ID-Nr. 1 oder Seq.-ID-Nr. 3 aufweist.

**7.** Test auf Endotoxin, umfassend:

i) das Kontaktieren einer zu testenden Probe, die ein Peptid umfasst, das spaltbar ist, um eine chromogene oder fluorogene Gruppierung zu erzeugen, mit einem enzymatisch aktiven rekombinanten Faktor C eines Molukkenkrebses, der durch die Expression eines rekombinanten DNA-Vektors nach einem der Ansprüche 1 bis 6 in einer Insektenwirtszellkultur erhalten wird; und
ii) das Messen der Menge der vom Peptid abgespaltenen chromogenen oder fluorogenen Gruppierung.

**8.** Test nach Anspruch 7, worin das Peptid N-t-Boc-Val-Pro-Arg-MCA, Mu-Val-Pro-Arg-AFC oder Boc-Val-Pro-Arg-pNA ist.

**9.** Test nach Anspruch 7 oder 8, worin die Insektenwirtszelle eine Schmetterlingszelle ist.

**10.** Test auf Endotoxin, umfassend:

(i) das Kontaktieren einer zu testenden Probe mit einem immobilisierten Antikörper, der spezifisch an Lipopolysaccharid bindet, oder einem immobilisierten Antikörper, der spezifisch an Lipid A bindet, um einen Komplex zwischen dem Antikörper und Endotoxin in der Probe zu bilden;
ii) das Kontaktieren des Komplexes mit einem enzymatisch aktiven rekombinanten Faktor C eines Molukkenkrebses, der durch die Expression eines rekombinanten DNA-Vektors nach einem der Ansprüche 1 bis 6 in einer Insektenwirtszellkultur erhältlich ist; und

iii) das Kontaktieren des immobilisierten Faktors C mit einem Antikörper, der spezifisch an den immobilisierten Faktor C bindet; und
iv) das Quantifizieren der Menge des spezifisch an den immobilisierten Faktor C gebundenen Antikörpers.

**11.** Test nach Anspruch 10, worin die Insektenwirtszelle eine Schmetterlingszelle ist.

**12.** Verfahren zum Entfernen von Endotoxin oder Lipid A aus einer Probe, umfassend:

i) das Kontaktieren von immobilisiertem rekombinantem Faktor C eines Molukkenkrebses mit der Probe, so dass Endotoxin oder Lipid A in der Probe an den immobilisierten rekombinanten Faktor C bindet; und
ii) das Abtrennen des immobilisierten rekombinanten Faktors C, an den das Endotoxin oder Lipid A gebunden ist, von der Probe;

worin der rekombinante Faktor C durch Expression eines rekombinanten DNA-Vektors nach einem der Ansprüche 1 bis 6 in einer Insektenwirtszellkultur produziert wird.

**13.** Rekombinanter DNA-Vektor, umfassend (i) einen von Baculovirus abgeleiteten Vektor oder einen Vektor zur Expression von heterologer DNA in einer Insektenzelllinie und (ii) eine Nucleinsäure, die für ein Polypeptid kodiert, das zu 75 % oder mehr Sequenzidentität mit einer Aminosäuresequenz aufweist, die aus der aus Seq.-ID-Nr. 2, Seq.-ID-Nr. 4 und der Aminosäuresequenz von Faktor C von Tachypleus tridentatus bestehenden Gruppe ausgewählt ist, worin der rekombinante DNA-Vektor zur Expression von enzymatisch aktivem Faktor C eines Molukkenkrebses führt.

**14.** Rekombinanter DNA-Vektor nach Anspruch 13, worin die Nucleinsäure (ii) von einem Molukkenkrebs des Genus Limulus erhalten wird.

## Revendications

**1.** Vecteur d'ADN recombinant comprenant un vecteur dérivé de baculorivus et un ADNc codant pour un Facteur C enzymatiquement actif d'un crabe des Moluques.

**2.** Vecteur de la revendication 1, où ledit vecteur dérivé de baculovirus est pFastBacI.

**3.** Vecteur de la revendication 2, qui est pFastBac/CrFC21.

**4.** Vecteur de la revendication 1, où ledit ADNc codant pour un Facteur C enzymatiquement actif comprend un polynucléotide qui s'hybride à une séquence de nucléotides sélectionnée parmi SEQ ID NO:1, le complément de SEQ ID NO:1, SEQ ID NO:3, le complément de SEQ ID NO:3 ou un ADNc de *Tachypleus tridentatus* codant pour une enzyme du Facteur C, en conditions stringentes.

**5.** Vecteur de la revendication 1, où ledit ADNc codant pour un Facteur C enzymatiquement actif comprend un polynucléotide qui code pour une séquence d'acides aminés sélectionnée parmi SEQ ID NO:2, SEQ ID NO:4, ou la séquence d'acides aminés du Facteur C de *Tachypleus tridentatus.*

**6.** Vecteur de la revendication 1, où ledit ADNc codant pour un Facteur C enzymatiquement actif comprend un polynucléotide ayant la séquence de SEQ ID NO:1 ou SEQ ID NO:3.

**7.** Essai pour l'endotoxine comprenant :

i) la mise en contact d'un échantillon à analyser, comprenant un peptide scindable pour produire une fraction chromogène ou fluorogène avec un Facteur C recombinant enzymatiquement actif d'un crabe des moluques obtenu par l'expression d'un vecteur d'ADN recombinant selon l'une quelconque des revendications 1 à 6 dans une culture de cellules hôtes d'insectes ;

ii) la mesure de la quantité de ladite fraction chromogène ou fluorogène scindée dudit peptide.

**8.** Essai de la revendication 7, où ledit peptide est N-t-Boc-Val-Pro-Arg-MCA, Mu-Val-Pro-Arg-AFC ou Boc-Val-Pro-Arg-pNA.

**9.** Essai de la revendication 7 ou 8, où ladite cellule hôte d'insecte est une cellule de lépidoptère.

**10.** Essai pour l'endotoxine comprenant :

(i) la mise en contact d'un échantillon à analyser avec un anticorps immobilisé qui se lie spécifiquement à un lipopolysaccharide ou un anticorps immobilisé qui se lie spécifiquement au lipide A pour former un complexe entre ledit anticorps et l'endotoxine dans ledit échantillon ;

(ii) la mise en contact dudit complexe avec un facteur recombinant enzymatiquement actif d'un crabe des Moluques pouvant être obtenu par expression d'un vecteur d'ADN recombinant selon l'une quelconque des revendications 1 à 6 dans une culture de cellules hôtes d'insectes ; et

(iii) la mise en contact dudit Facteur C immobilisé avec un anticorps qui se lie spécifiquement audit Facteur C immobilisé ; et

(iv) la quantification de la quantité dudit anticorps spécifiquement lié audit Facteur C immobilisé.

**11.** Essai de la revendication 10, où ladite cellule hôte d'insecte est une cellule de lépidoptère.

**12.** Méthode pour éliminer l'endotoxine ou un lipide A d'un échantillon comprenant :

i) la mise en contact du Facteur C recombinant immobilisé d'un crabe des Moluques avec ledit échantillon de façon que l'endotoxine ou le lipide A dans ledit échantillon se lie audit facteur C recombinant immobilisé ; et

ii) la séparation dudit Facteur C recombinant immobilisé ayant ladite endotoxine ou ledit lipide A qui lui est lié, dudit échantillon ;

où ledit Facteur C recombinant est produit par expression d'un vecteur d'ADN recombinant selon l'une quelconque des revendications 1 à 6 dans une culture de cellules hôtes d'insectes.

**13.** Vecteur d'ADN recombinant comprenant (i) un vecteur dérivé d'un baculovirus ou un vecteur pour l'expression d'un ADN hétérologue dans une lignée de cellules d'insectes et (ii) un acide nucléique codant pour un polypeptide ayant 75% d'identité de séquence ou plus avec une séquence d'acides aminés sélectionnée dans le groupe consistant en SEQ ID NO:2, SEQ ID NO:4 et la séquence d'acides aminés du Facteur C de *Tachypleus tridentatus*, où le vecteur d'ADN recombinant mène à l'expression d'un Facteur C enzymatiquement actif d'un crabe des Moluques.

**14.** Vecteur d'ADN recombinant de la revendication 13, où ledit acide nucléique (ii) est obtenu à partir d'un crabe des Moluques du genre *Limulus.*

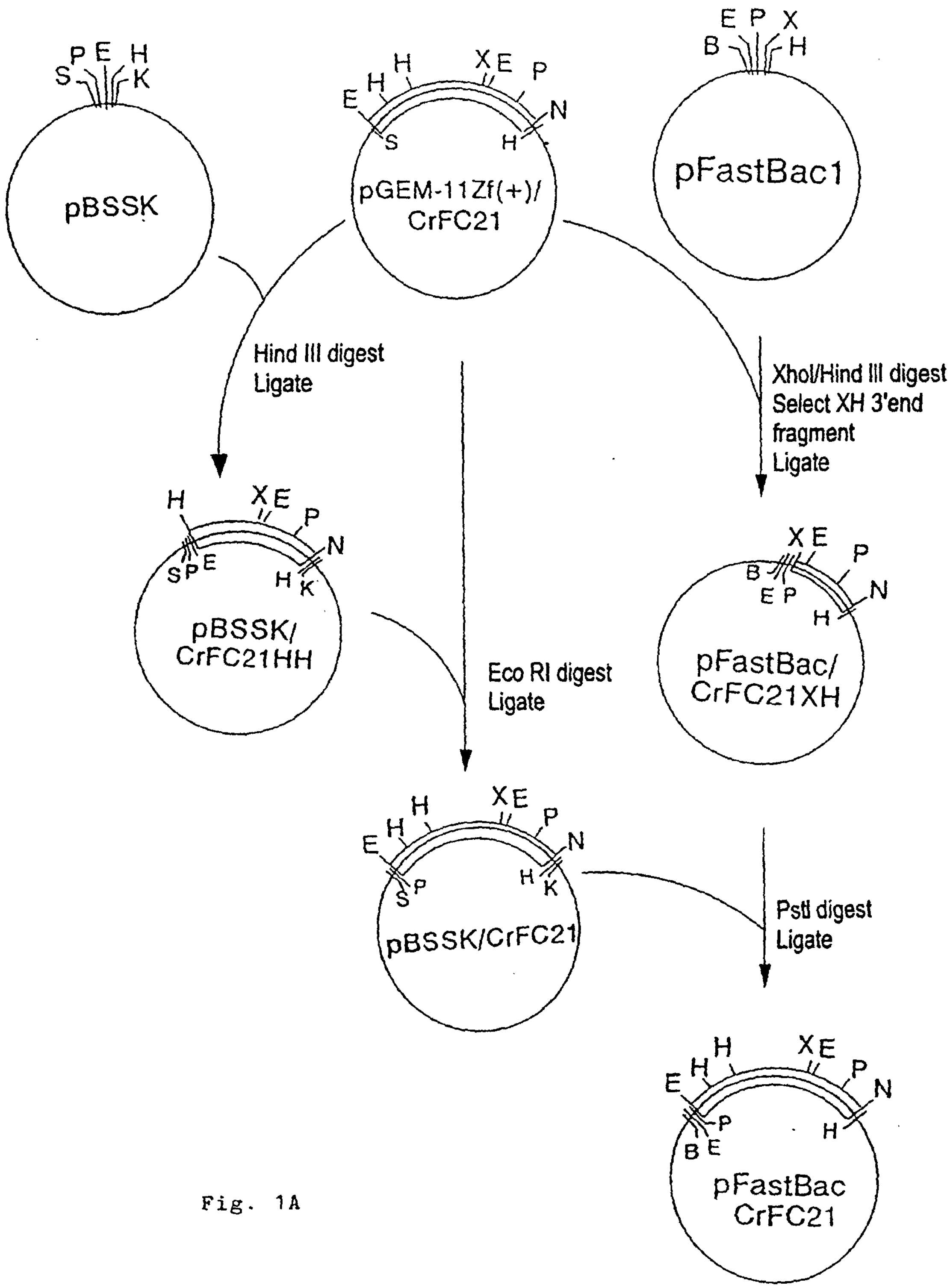
pBSSK
S
P
E
H
K
pGEM-11Zf(+)/
CrFC21
pFastBac1
B
E
P
X
H
Hind III digest
Ligate
Xhol/Hind III digest
Select XH 3'end
fragment
Ligate
pBSSK/
CrFC21HH
Eco RI digest
Ligate
pFastBac/
CrFC21XH
pBSSK/CrFC21
PstI digest
Ligate
pFastBac
CrFC21

Fig. 1A

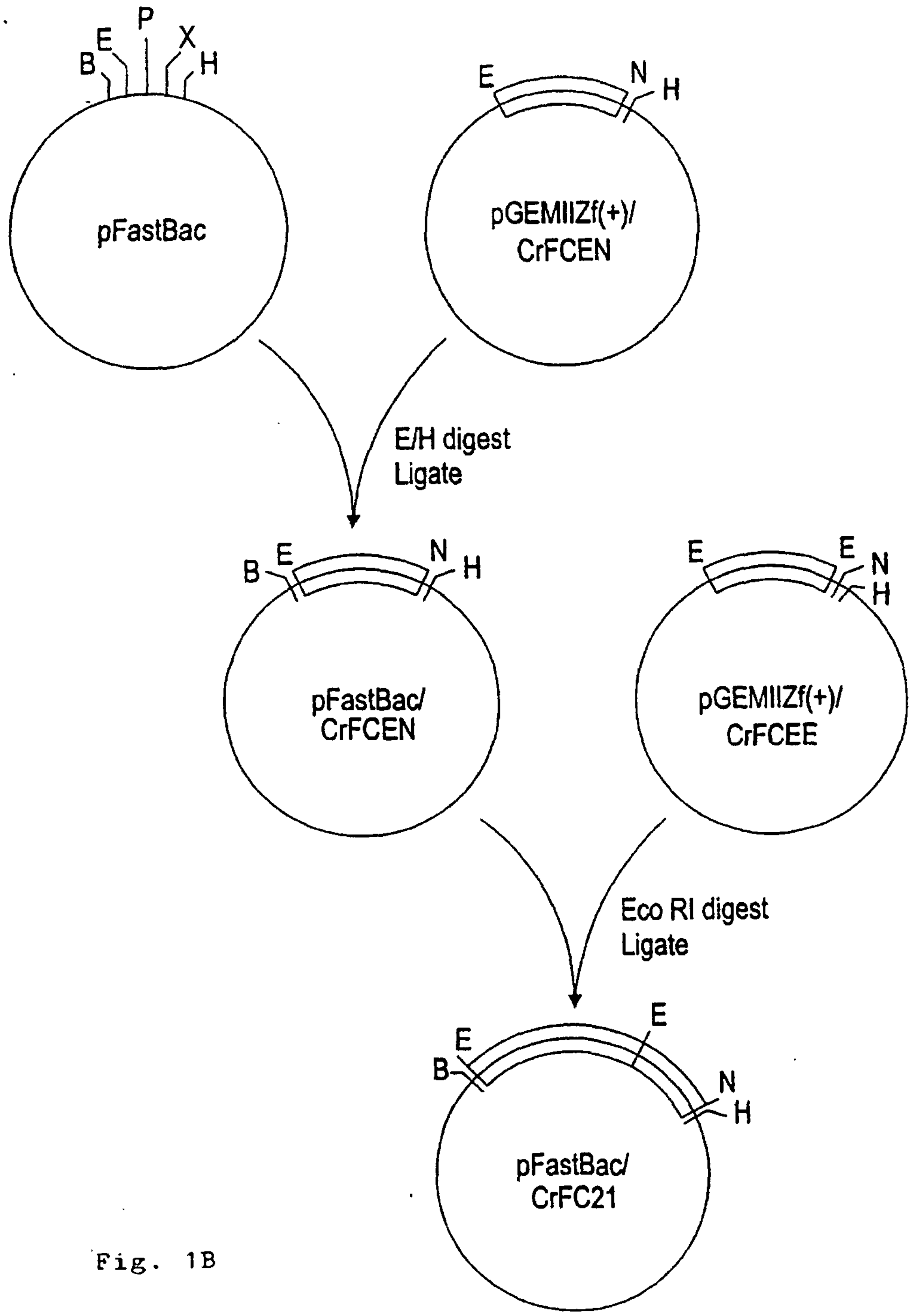
B
E
P
X
H
pFastBac
E
N
H
pGEMIIZf(+)/
CrFCEN
E/H digest
Ligate
B
E
N
H
pFastBac/
CrFCEN
E
E
N
H
pGEMIIZf(+)/
CrFCEE
Eco RI digest
Ligate
B
E
E
N
H
pFastBac/
CrFC21

Fig. 1B

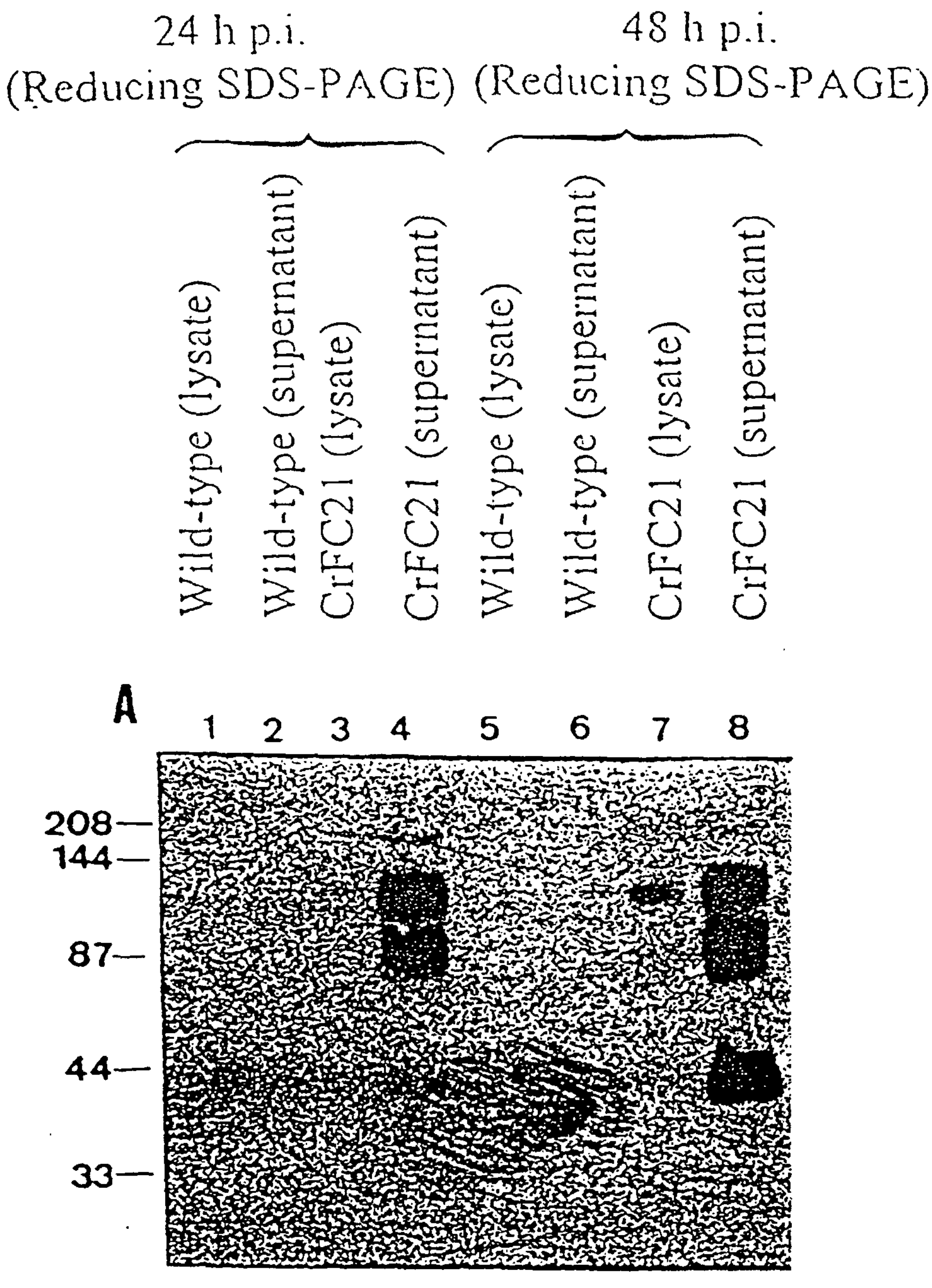
24 h p.i.
(Reducing SDS-PAGE)
48 h p.i.
(Reducing SDS-PAGE)
Wild-type (lysate)
Wild-type (supernatant)
CrFC21 (lysate)
CrFC21 (supernatant)
Wild-type (lysate)
Wild-type (supernatant)
CrFC21 (lysate)
CrFC21 (supernatant)
A
1
2
3
4
5
6
7
8
208
144
87
44
33

Fig. 2A

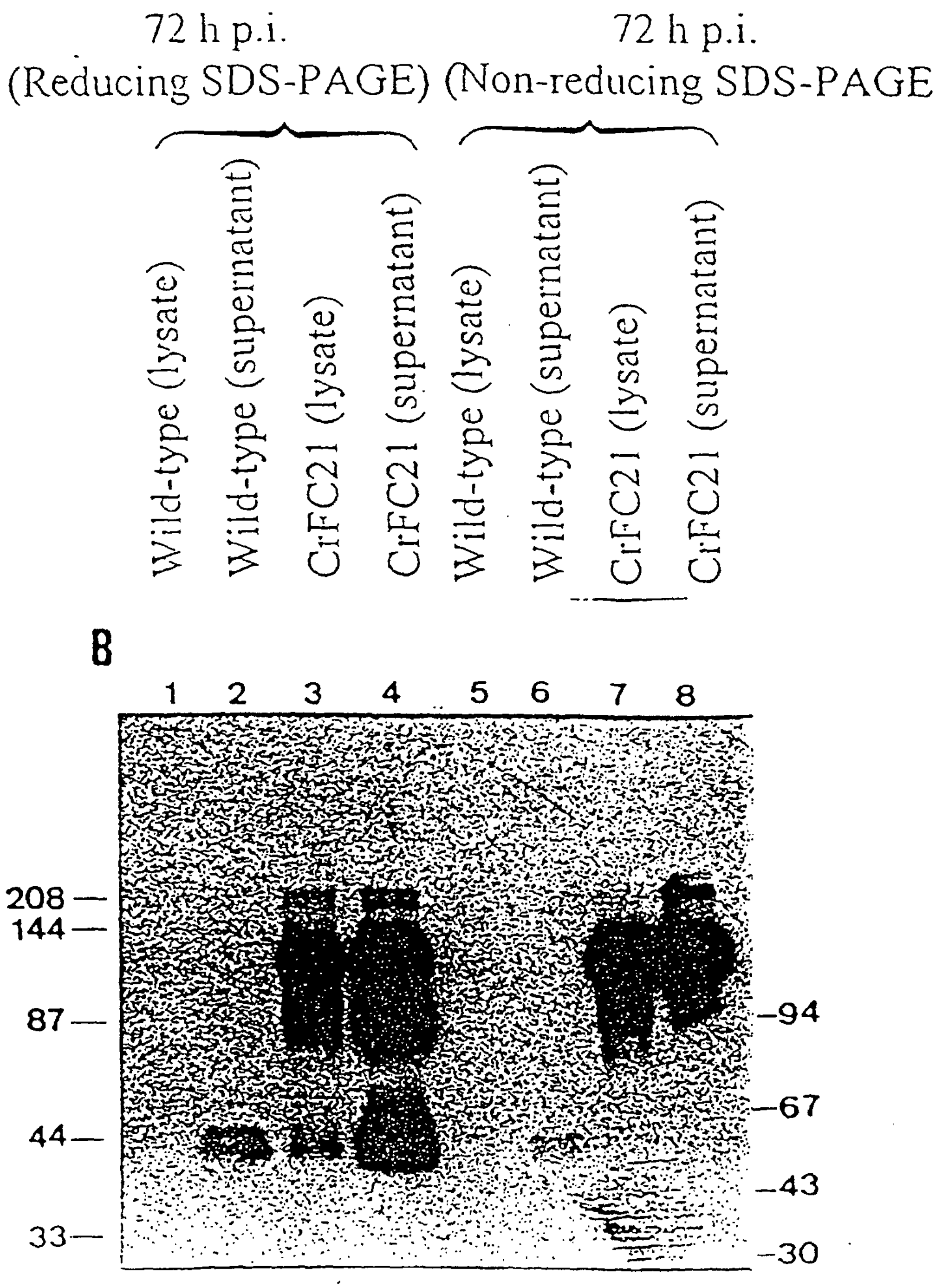
72 h p.i.
(Reducing SDS-PAGE)
72 h p.i.
(Non-reducing SDS-PAGE
Wild-type (lysate)
Wild-type (supernatant)
CrFC21 (lysate)
CrFC21 (supernatant)
Wild-type (lysate)
Wild-type (supernatant)
CrFC21 (lysate)
CrFC21 (supernatant)
B
1 2 3 4 5 6 7 8
208
144
87
44
33
94
67
43
30

Fig. 2B

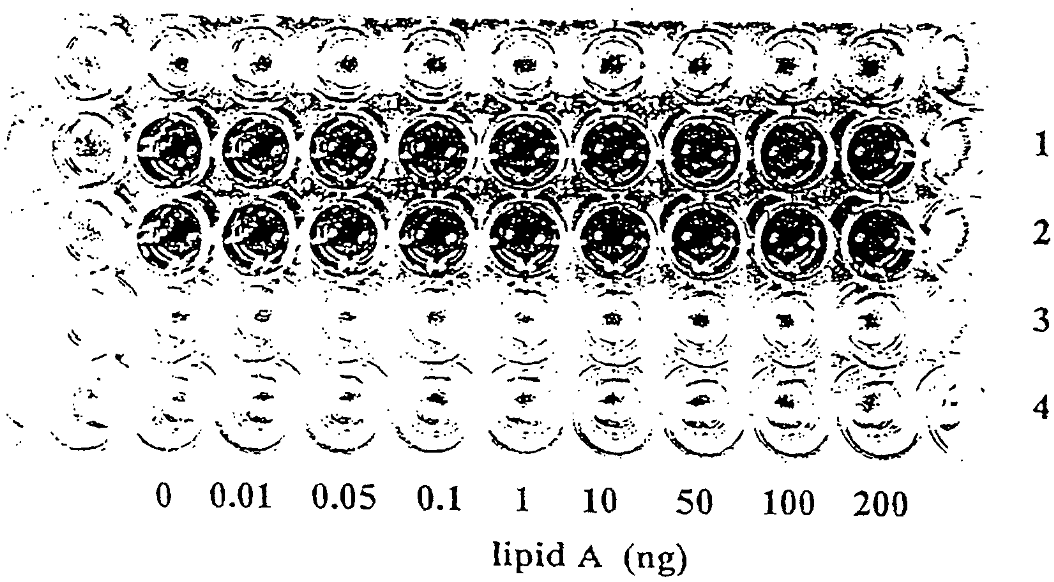


Fig. 3A

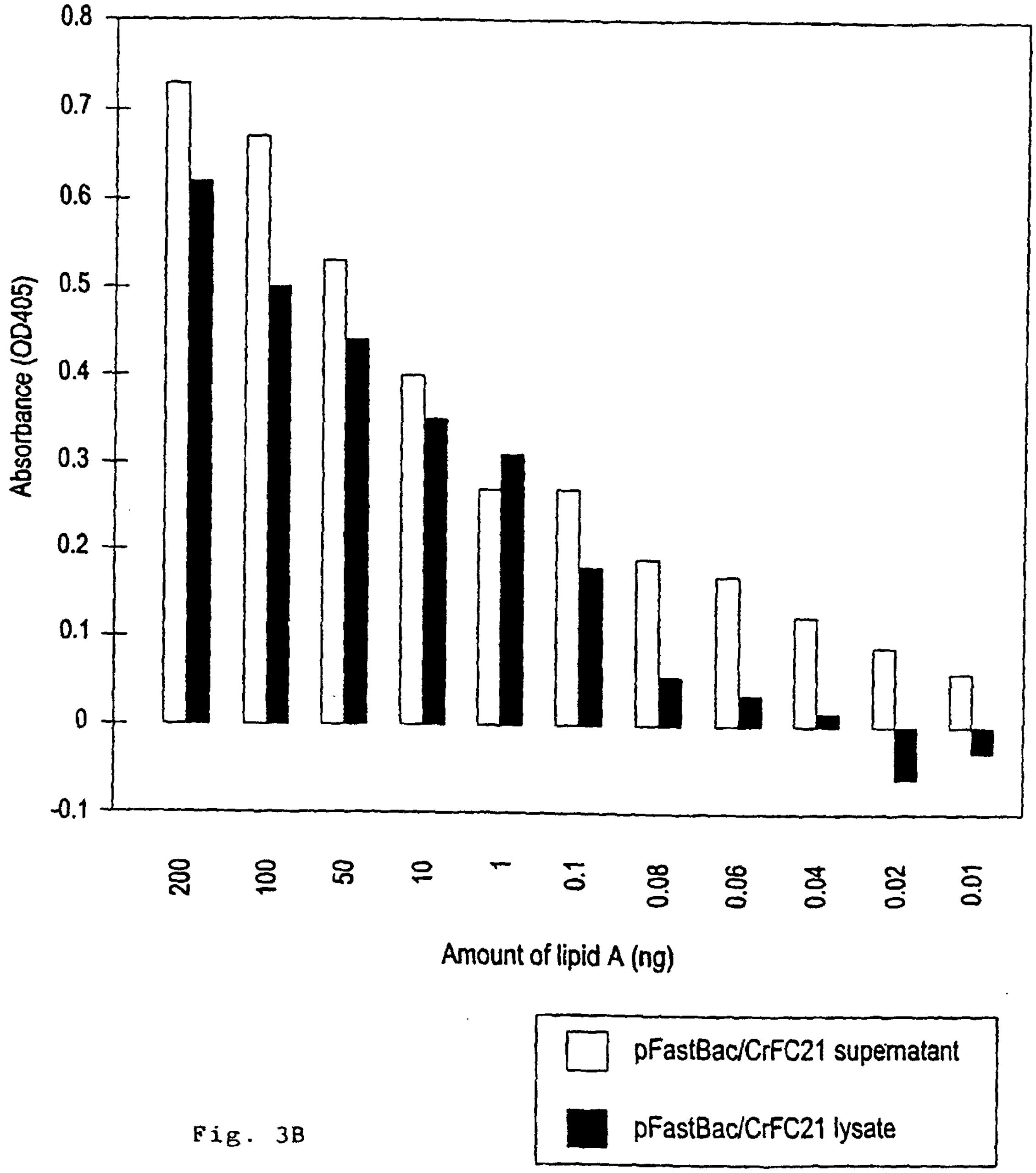
0.8
0.7
0.6
0.5
0.4
0.3
0.2
0.1
0
-0.1
Absorbance (OD405)
200
100
50
10
1
0.1
0.08
0.06
0.04
0.02
0.01
Amount of lipid A (ng)
pFastBac/CrFC21 supernatant
pFastBac/CrFC21 lysate

Fig. 3B

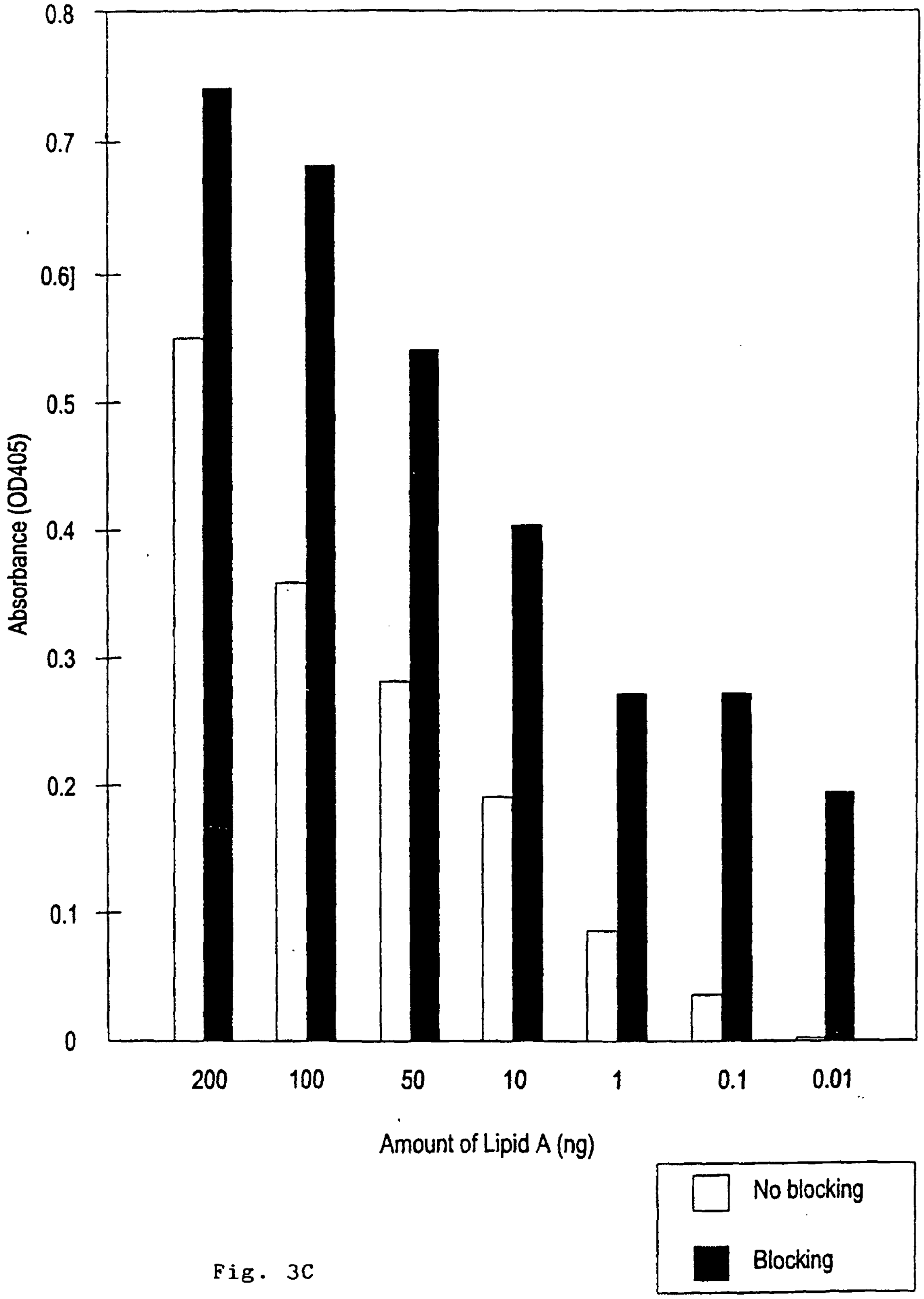
0.8
0.7
0.6]
0.5
0.4
0.3
0.2
0.1
0
Absorbance (OD405)
200
100
50
10
1
0.1
0.01
Amount of Lipid A (ng)
No blocking
Blocking

Fig. 3C

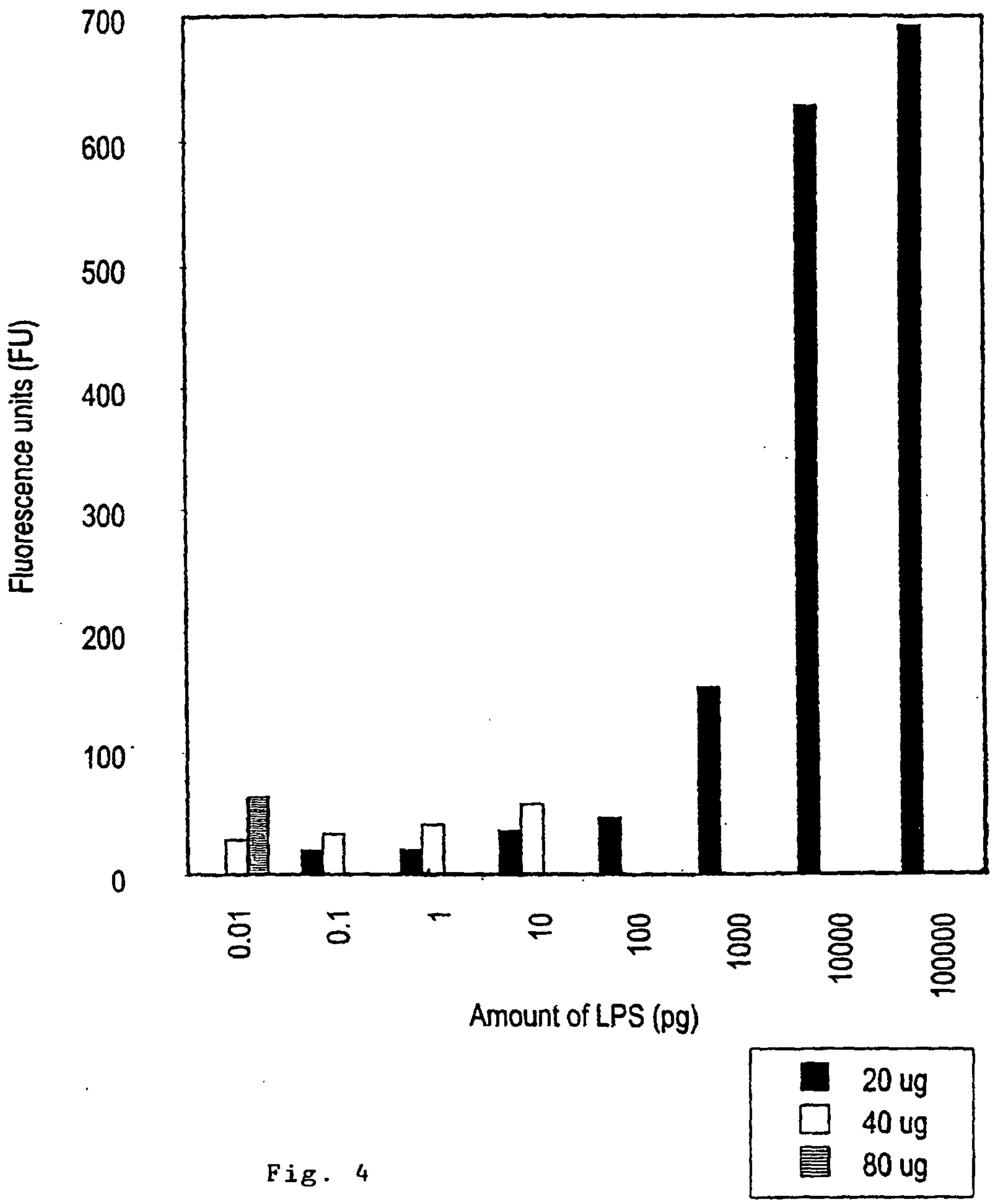

700
600
500
400
300
200
100
0
Fluorescence units (FU)
0.01
0.1
1
10
100
1000
10000
100000
Amount of LPS (pg)
20 ug
40 ug
80 ug


Fig. 4

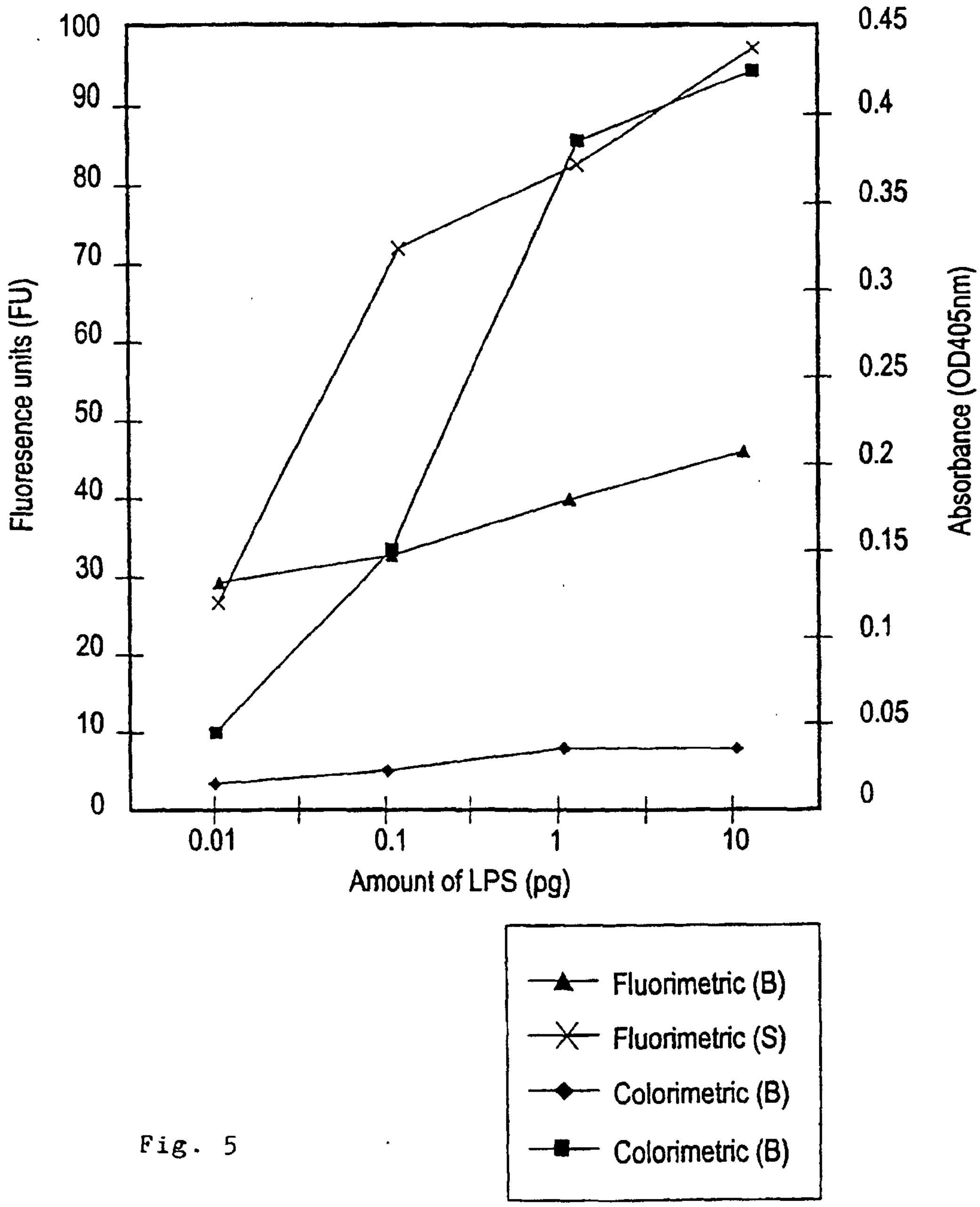
100
90
80
70
60
50
40
30
20
10
0
0.45
0.4
0.35
0.3
0.25
0.2
0.15
0.1
0.05
0
Fluoresence units (FU)
Absorbance (OD405nm)
0.01
0.1
1
10
Amount of LPS (pg)
Fluorimetric (B)
Fluorimetric (S)
Colorimetric (B)
Colorimetric (B)

Fig. 5

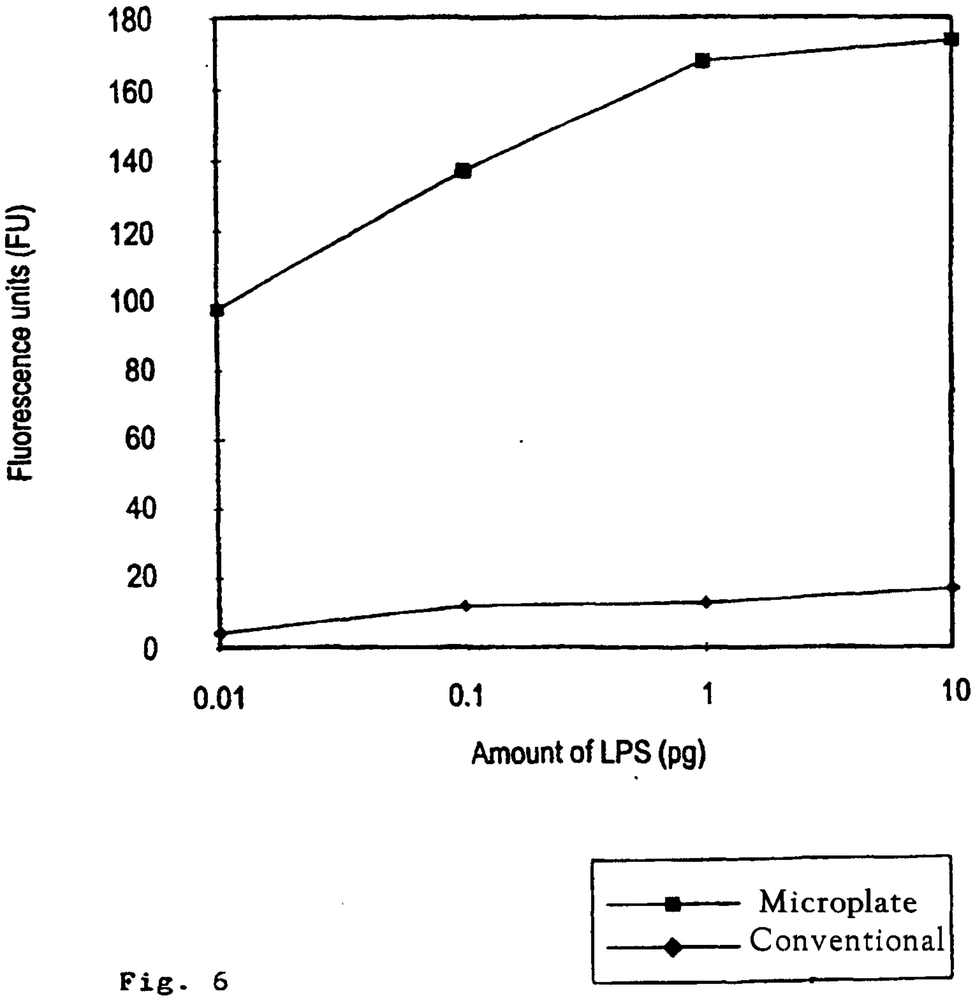
180
160
140
120
100
80
60
40
20
0
Fluorescence units (FU)
0.01
0.1
1
10
Amount of LPS (pg)
Microplate
Conventional

Fig. 6

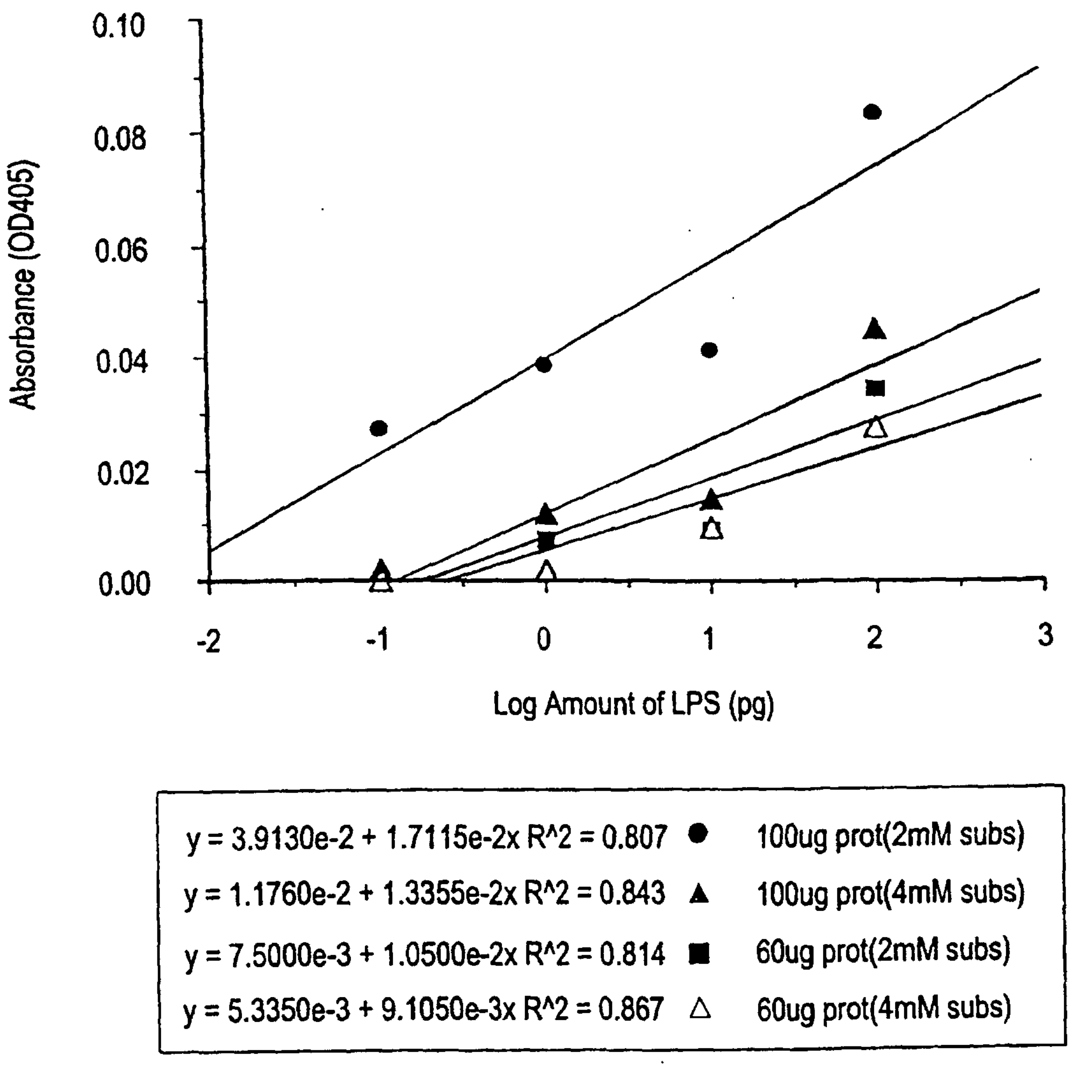
0.10
0.08
0.06
0.04
0.02
0.00
Absorbance (OD405)
-2
-1
0
1
2
3
Log Amount of LPS (pg)
y = 3.9130e-2 + 1.7115e-2x R^2 = 0.807 ● 100ug prot(2mM subs)
y = 1.1760e-2 + 1.3355e-2x R^2 = 0.843 ▲ 100ug prot(4mM subs)
y = 7.5000e-3 + 1.0500e-2x R^2 = 0.814 ■ 60ug prot(2mM subs)
y = 5.3350e-3 + 9.1050e-3x R^2 = 0.867 △ 60ug prot(4mM subs)

Fig. 7

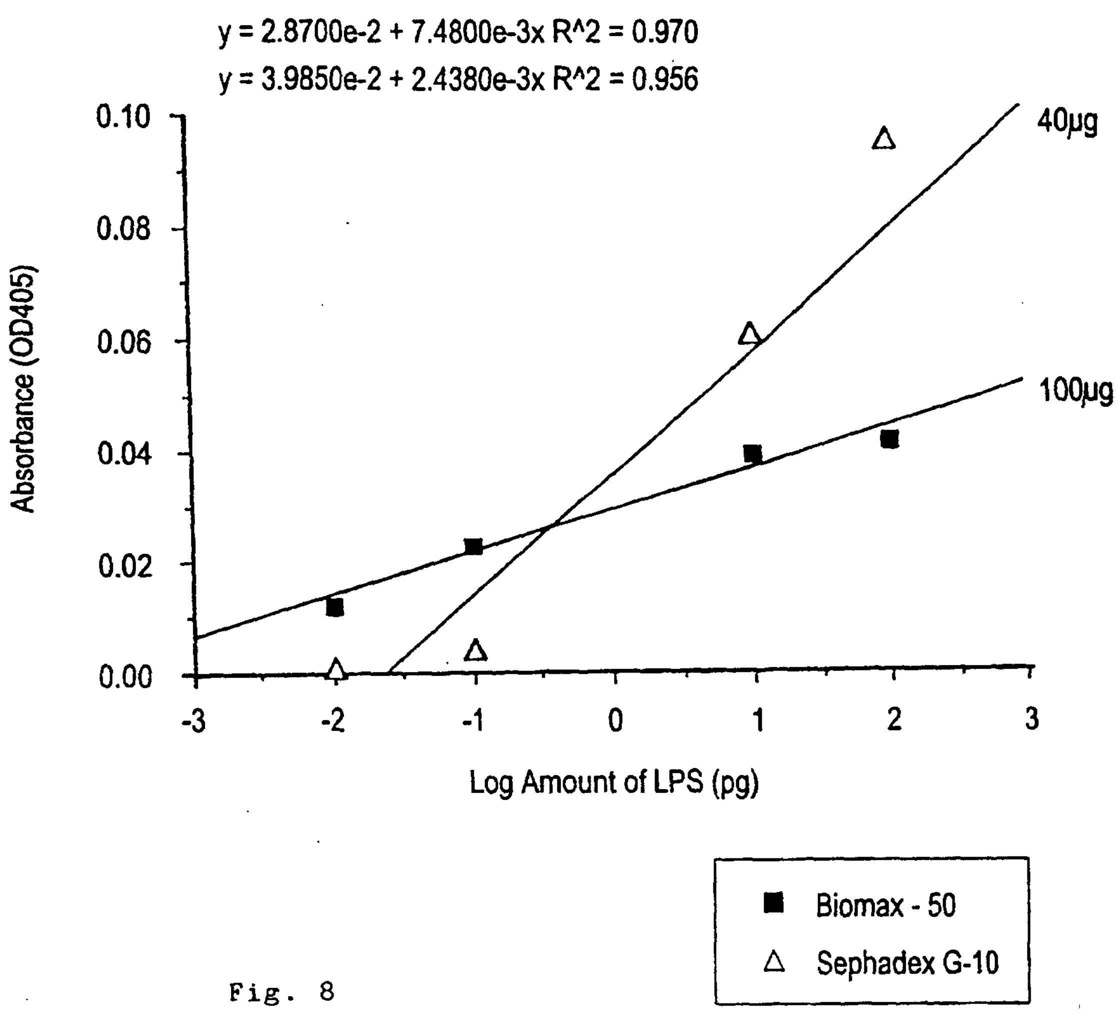
y = 2.8700e-2 + 7.4800e-3x R^2 = 0.970
y = 3.9850e-2 + 2.4380e-3x R^2 = 0.956
0.10
0.08
0.06
0.04
0.02
0.00
Absorbance (OD405)
-3
-2
-1
0
1
2
3
Log Amount of LPS (pg)
40µg
100µg
Biomax - 50
Sephadex G-10

Fig. 8

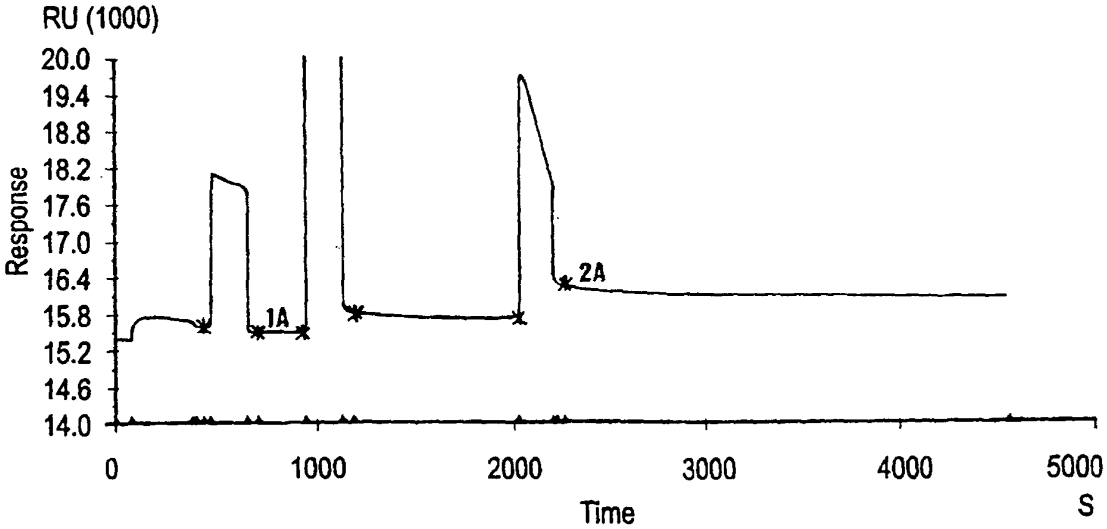


| Fc | Time | AbsResp | Slope | LRSD | Baseline | RelResp | Ld |
|---|---|---|---|---|---|---|---|
| 1 | 445.5 | 15605.1 | -1.30 | 0.86 | No | #N/A | before w/t |
| 1 | 713.5 | 15509.4 | -3.51 | 4.15 | No | #N/A | w/t |
| 1 | 936.5 | 15484.9 | -0.16 | 0.18 | No | #N/A | before w/t2x |
| 1 | 1197.5 | 15798.6 | -1.62 | 0.26 | No | #N/A | w/t2x |
| 1 | 2023.5 | 15728.3 | -0.46 | 0.82 | No | #N/A | before rFCSf9 |
| 1 | 2268.5 | 16281.2 | -3.28 | 0.62 | No | #N/A | rFCSf9 |

Fig. 9

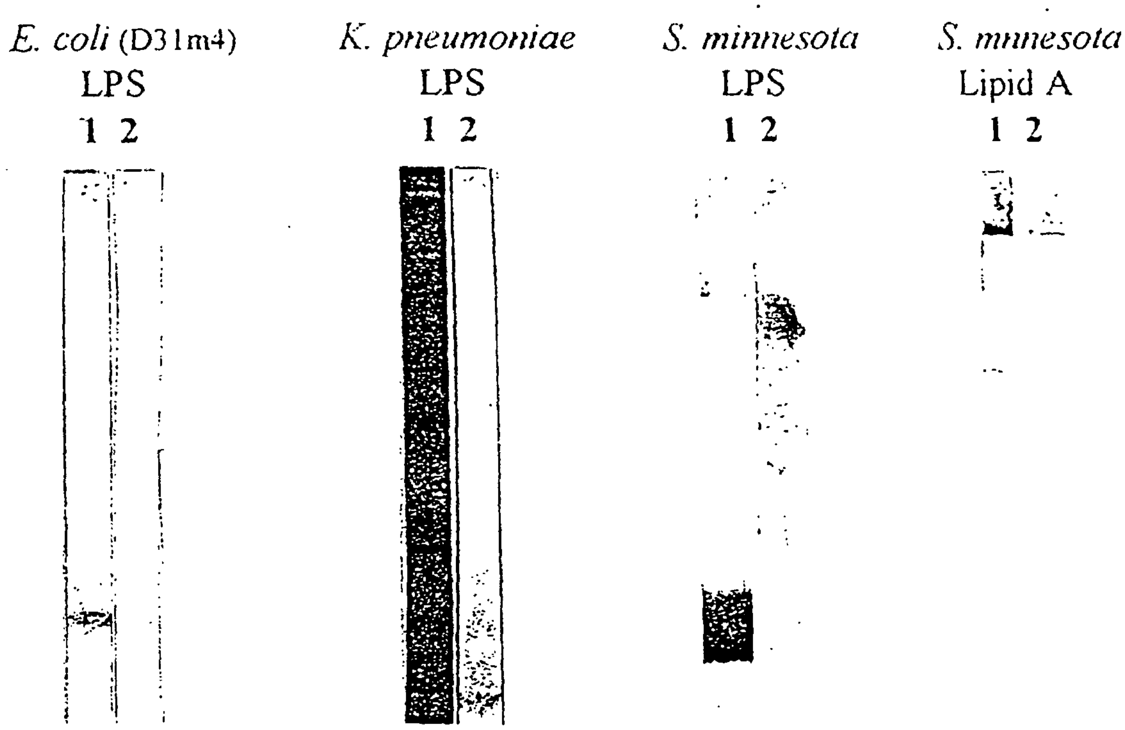
*E. coli* (D31m4)
LPS
1 2
*K. pneumoniae*
LPS
1 2
*S. minnesota*
LPS
1 2
*S. mnnesota*
Lipid A
1 2

Fig. 10

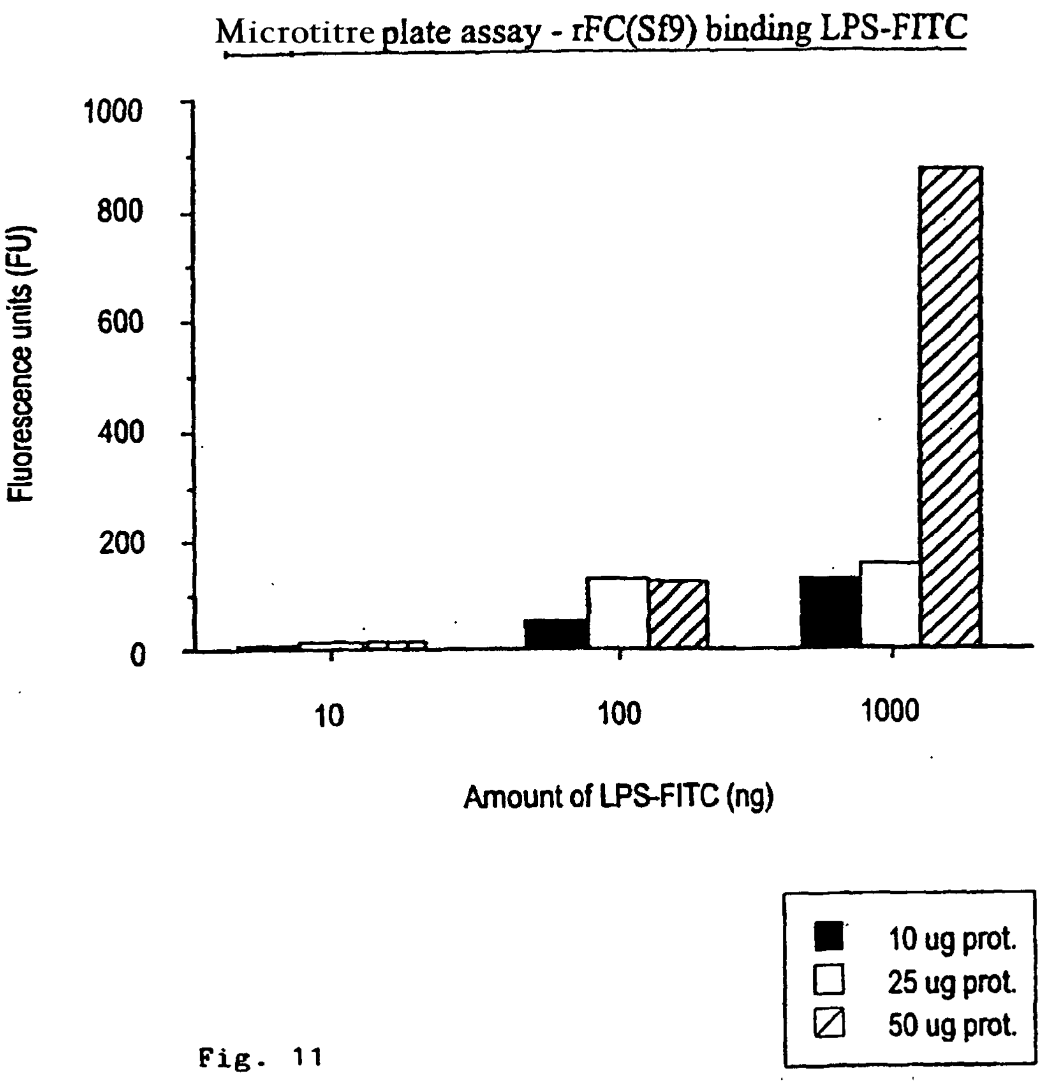
Microtitre plate assay - rFC(Sf9) binding LPS-FITC
1000
800
600
400
200
0
Fluorescence units (FU)
10
100
1000
Amount of LPS-FITC (ng)
10 ug prot.
25 ug prot.
50 ug prot.

Fig. 11

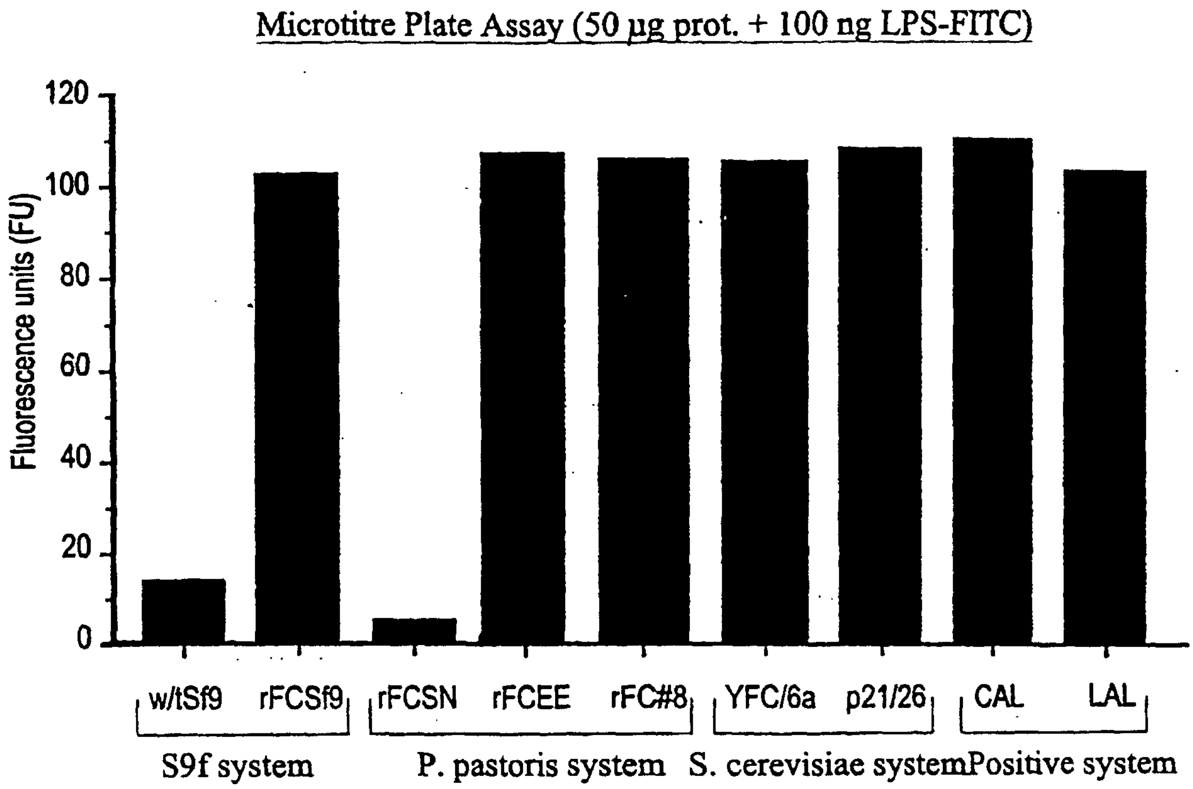
Microtitre Plate Assay (50 µg prot. + 100 ng LPS-FITC)
Fluorescence units (FU)
120
100
80
60
40
20
0
w/tSf9
rFCSf9
rFCSN
rFCEE
rFC#8
YFC/6a
p21/26
CAL
LAL
S9f system
P. pastoris system
S. cerevisiae system
Positive system

Fig. 12

SEQUENCE LISTING

(1) GENERAL INFORMATION:

(i) APPLICANT:

(ii) TITLE OF INVENTION: Cloning and Expression of *Carcinoscorpius rotundicauda* Factor C in a Baculoviral Host-vector System

(iii) NUMBER OF SEQUENCES: 4

(iv) CORRESPONDENCE ADDRESS:

(A) ADDRESSEE: Birch, Stewart, Kolasch & Birch
(B) STREET: 8110 Gatehouse Road, Suite 500 East
(C) CITY: Falls Church
(D) STATE: Virginia
(E) COUNTRY: USA
(F) ZIP: 22042

(v) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: Patent In Release #1.0, Version #1.25

(vi) CURRENT APPLICATION DATA:

(A) APPLICATION NUMBER: US
(B) FILING DATE:
(C) CLASSIFICATION:

(viii) ATTORNEY/AGENT INFORMATION:

(A) NAME: Murphy, Jr., Gerald M.
(B) REGISTRATION NUMBER: 28,977
(C) REFERENCE/DOCKET NUMBER: 1781-105P

(ix) TELECOMMUNICATION INFORMATION:

(A) TELEPHONE: (703) 205-8000
(B) TELEFAX: (703) 205-8050
(C) TELEX: 248345

(2) INFORMATION FOR SEQ ID NO:1:

(i) SEQUENCE CHARACTERISTICS:

(A) LENGTH: 4182 base pairs
(B) TYPE: nucleic acid

(C) STRANDEDNESS: single
(D) TOPOLOGY: both

(ii) MOLECULE TYPE: cDNA

(iii) HYPOTHETICAL: NO

(iv) ANTI-SENSE: NO

(vi) ORIGINAL SOURCE:

(A) ORGANISM: Carcinoscorpius rotundicauda

(ix) FEATURE:

(A) NAME/KEY: CDS
(B) LOCATION: 569..3817

(xi) SEQUENCE DESCRIPTION: SEQ ID NO:1:

```
GTATTTAATG TCTCAACGGT AAAGGTTTCA TTGTAGCTAA TATTTAACTT CCTCCCTGTG       60

CCCCAAATCG CGAGTATGAC GTCAGTTAAG ACTTCGTATT TTAAGAGTTA AACACGAGCC      120

TTAAAGAGCG ATATTTTTTT TGTTAAACAC TTCCAACTTA ATACAATTGG CAAACTTTCA      180

AAAATAAAGT GGAAAAGGAG GTAAAAAAGA TGAAAAAAAT TCGCATACAA TAGAATACAA      240

TAAAATGTGT TGTCTTTACT GTCAACACTT ACTGTTCGTT CGGTCACAGC TGTGAATCGG      300

GGTGACTTTA TGTTTGTAGT GGTCTTAAAA ACGGGTACTT GGTTGTTTTG AAAATTTTAA      360

AACCTACATA TGATTCTCCT AAAATTTTGT TTATAAATTA GCACCATTTG CGACCTAAAT      420

CTTTTTTGTA GTCTTAAGTT TAGTTGACAT AAAAACAAAA TTTGTAACAA CACACGGTAT      480

AAACTAAATA GCTTCAGATG GGTCGTATGA CAAGGAAACT TTTAAATAAT TATGAAAGTT      540

TTTTTAAAAT TTGACTAAGG TTTAGATT ATG TGG GTG ACA TGC TTC GAC ACG         592
                               Met Trp Val Thr Cys Phe Asp Thr
                                1               5

TTT CTT TTT GTT TGT GAA AGT TCA GTT TTC TGT TTG TTG TGT GTG TGG        640
Phe Leu Phe Val Cys Glu Ser Ser Val Phe Cys Leu Leu Cys Val Trp
    10                  15                  20

AGG TTT GGT TTC TGT AGG TGG CGT GTT TTC TAC AGT TTT CCA TTC GTT        688
Arg Phe Gly Phe Cys Arg Trp Arg Val Phe Tyr Ser Phe Pro Phe Val
 25                  30                  35                  40

AAG TCA ACA GTT GTT TTA TTA CAG TGT TAC CAT TAC TCT CTC CAC AAT        736
Lys Ser Thr Val Val Leu Leu Gln Cys Tyr His Tyr Ser Leu His Asn
                 45                  50                  55

ACC TCA AAG TTC TAC TCT GTG AAT CCT GAC AAG CCA GAG TAC ATT CTT        784
Thr Ser Lys Phe Tyr Ser Val Asn Pro Asp Lys Pro Glu Tyr Ile Leu
             60                  65                  70

TCA GGT TTA GTT CTA GGG CTA CTA GCC CAA AAA ATG CGC CCA GTT CAG        832
Ser Gly Leu Val Leu Gly Leu Leu Ala Gln Lys Met Arg Pro Val Gln
         75                  80                  85

TCC AAA GGA GTA GAT CTA GGC TTG TGT GAT GAA ACG AGG TTC GAG TGT        880
Ser Lys Gly Val Asp Leu Gly Leu Cys Asp Glu Thr Arg Phe Glu Cys
     90                  95                 100

AAG TGT GGC GAT CCA GGC TAT GTG TTC AAC ATT CCA GTG AAA CAA TGT        928
Lys Cys Gly Asp Pro Gly Tyr Val Phe Asn Ile Pro Val Lys Gln Cys
105                 110                 115                 120

ACA TAC TTT TAT CGA TGG AGG CCG TAT TGT AAA CCA TGT GAT GAC CTG        976
Thr Tyr Phe Tyr Arg Trp Arg Pro Tyr Cys Lys Pro Cys Asp Asp Leu
                125                 130                 135
```

```
GAG GCT AAG GAT ATT TGT CCA AAG TAC AAA CGA TGT CAA GAG TGT AAG        1024
Glu Ala Lys Asp Ile Cys Pro Lys Tyr Lys Arg Cys Gln Glu Cys Lys
            140                 145                 150

GCT GGT CTT GAT AGT TGT GTT ACT TGT CCA CCT AAC AAA TAT GGT ACT        1072
Ala Gly Leu Asp Ser Cys Val Thr Cys Pro Pro Asn Lys Tyr Gly Thr
        155                 160                 165

TGG TGT AGC GGT GAA TGT CAG TGT AAG AAT GGA GGT ATC TGT GAC CAG        1120
Trp Cys Ser Gly Glu Cys Gln Cys Lys Asn Gly Gly Ile Cys Asp Gln
    170                 175                 180

AGG ACA GGA GCT TGT GCA TGT CGT GAC AGA TAT GAA GGG GTG CAC TGT        1168
Arg Thr Gly Ala Cys Ala Cys Arg Asp Arg Tyr Glu Gly Val His Cys
185                 190                 195                 200

GAA ATT CTC AAA GGT TGT CCT CTT CTT CCA TCG GAT TCT CAG GTT CAG        1216
Glu Ile Leu Lys Gly Cys Pro Leu Leu Pro Ser Asp Ser Gln Val Gln
                205                 210                 215

GAA GTC AGA AAT CCA CCA GAT AAT CCC CAA ACT ATT GAC TAC AGC TGT        1264
Glu Val Arg Asn Pro Pro Asp Asn Pro Gln Thr Ile Asp Tyr Ser Cys
            220                 225                 230

TCA CCA GGG TTC AAG CTT AAG GGT ATG GCA CGA ATT AGC TGT CTC CCA        1312
Ser Pro Gly Phe Lys Leu Lys Gly Met Ala Arg Ile Ser Cys Leu Pro
        235                 240                 245

AAT GGA CAG TGG AGT AAC TTT CCA CCC AAA TGT ATT CGA GAA TGT GCC        1360
Asn Gly Gln Trp Ser Asn Phe Pro Pro Lys Cys Ile Arg Glu Cys Ala
    250                 255                 260

ATG GTT TCA TCT CCA GAA CAT GGG AAA GTG AAT GCT CTT AGT GGT GAT        1408
Met Val Ser Ser Pro Glu His Gly Lys Val Asn Ala Leu Ser Gly Asp
265                 270                 275                 280

ATG ATA GAA GGG GCT ACT TTA CGG TTC TCA TGT GAT AGT CCC TAC TAC        1456
Met Ile Glu Gly Ala Thr Leu Arg Phe Ser Cys Asp Ser Pro Tyr Tyr
                285                 290                 295

TTG ATT GGT CAA GAA ACA TTA ACC TGT CAG GGT AAT GGT CAG TGG AAT        1504
Leu Ile Gly Gln Glu Thr Leu Thr Cys Gln Gly Asn Gly Gln Trp Asn
            300                 305                 310

GGA CAG ATA CCA CAA TGT AAG AAC TTA GTC TTC TGT CCT GAC CTG GAT        1552
Gly Gln Ile Pro Gln Cys Lys Asn Leu Val Phe Cys Pro Asp Leu Asp
        315                 320                 325

CCT GTA AAC CAT GCT GAA CAC AAG GTT AAA ATT GGT GTG GAA CAA AAA        1600
Pro Val Asn His Ala Glu His Lys Val Lys Ile Gly Val Glu Gln Lys
    330                 335                 340

TAT GGT CAG TTT CCT CAA GGC ACT GAA GTG ACC TAT ACG TGT TCG GGT        1648
Tyr Gly Gln Phe Pro Gln Gly Thr Glu Val Thr Tyr Thr Cys Ser Gly
345                 350                 355                 360

AAC TAC TTC TTG ATG GGT TTT GAC ACC TTA AAA TGT AAC CCT GAT GGG        1696
Asn Tyr Phe Leu Met Gly Phe Asp Thr Leu Lys Cys Asn Pro Asp Gly
                365                 370                 375
```

TCT TGG TCA GGA TCA CAG CCA TCC TGT GTT AAA GTG GCA GAC AGA GAG 1744
Ser Trp Ser Gly Ser Gln Pro Ser Cys Val Lys Val Ala Asp Arg Glu
380 385 390

GTC GAC TGT GAC AGT AAA GCT GTA GAC TTC TTG GAT GAT GTT GGT GAA 1792
Val Asp Cys Asp Ser Lys Ala Val Asp Phe Leu Asp Asp Val Gly Glu
395 400 405

CCT GTC AGG ATC CAC TGT CCT GCT GGC TGT TCT TTG ACA GCT GGT ACT 1840
Pro Val Arg Ile His Cys Pro Ala Gly Cys Ser Leu Thr Ala Gly Thr
410 415 420

GTG TGG GGT ACA GCC ATA TAC CAT GAA CTT TCC TCA GTG TGT CGT GCA 1888
Val Trp Gly Thr Ala Ile Tyr His Glu Leu Ser Ser Val Cys Arg Ala
425 430 435 440

GCC ATC CAT GCT GGC AAG CTT CCA AAC TCT GGA GGA GCG GTG CAT GTT 1936
Ala Ile His Ala Gly Lys Leu Pro Asn Ser Gly Gly Ala Val His Val
445 450 455

GTG AAC AAT GGC CCC TAC TCG GAC TTT CTG GGT AGT GAC CTG AAT GGG 1984
Val Asn Asn Gly Pro Tyr Ser Asp Phe Leu Gly Ser Asp Leu Asn Gly
460 465 470

ATA AAA TCC GAA GAG TTG AAG TCT CTT GCC CGG AGT TTC CGA TTC GAT 2032
Ile Lys Ser Glu Glu Leu Lys Ser Leu Ala Arg Ser Phe Arg Phe Asp
475 480 485

TAT GTC AGT TCC TCC ACA GCA GGT AAA TCA GGA TGT CCT GAT GGA TGG 2080
Tyr Val Ser Ser Ser Thr Ala Gly Lys Ser Gly Cys Pro Asp Gly Trp
490 495 500

TTT GAG GTA GAC GAG AAC TGT GTG TAC GTT ACA TCA AAA CAG AGA GCC 2128
Phe Glu Val Asp Glu Asn Cys Val Tyr Val Thr Ser Lys Gln Arg Ala
505 510 515 520

TGG GAA AGA GCT CAA GGT GTG TGT ACC AAT ATG GCT GCT CGT CTT GCT 2176
Trp Glu Arg Ala Gln Gly Val Cys Thr Asn Met Ala Ala Arg Leu Ala
525 530 535

GTG CTG GAC AAA GAT GTA ATT CCA AAT TCA TTG ACT GAG ACT CTA CGA 2224
Val Leu Asp Lys Asp Val Ile Pro Asn Ser Leu Thr Glu Thr Leu Arg
540 545 550

GGG AAA GGG TTA ACA ACC ACG TGG ATA GGA TTG CAC AGA CTA GAT GCT 2272
Gly Lys Gly Leu Thr Thr Thr Trp Ile Gly Leu His Arg Leu Asp Ala
555 560 565

GAG AAG CCC TTT ATT TGG GAG TTA ATG GAT CGT AGT AAT GTG GTT CTG 2320
Glu Lys Pro Phe Ile Trp Glu Leu Met Asp Arg Ser Asn Val Val Leu
570 575 580

AAT GAT AAC CTA ACA TTC TGG GCC TCT GGC GAA CCT GGA AAT GAA ACT 2368
Asn Asp Asn Leu Thr Phe Trp Ala Ser Gly Glu Pro Gly Asn Glu Thr
585 590 595 600

AAC TGT GTA TAT ATG GAC ATC CAA GAT CAG TTG CAG TCT GTG TGG AAA 2416
Asn Cys Val Tyr Met Asp Ile Gln Asp Gln Leu Gln Ser Val Trp Lys
605 610 615

```
ACC AAG TCA TGT TTT CAG CCC TCA AGT TTT GCT TGC ATG ATG GAT CTG      2464
Thr Lys Ser Cys Phe Gln Pro Ser Ser Phe Ala Cys Met Met Asp Leu
            620                 625                 630

TCA GAC AGA AAT AAA GCC AAA TGC GAT GAT CCT GGA TCA CTG GAA AAT      2512
Ser Asp Arg Asn Lys Ala Lys Cys Asp Asp Pro Gly Ser Leu Glu Asn
        635                 640                 645

GGA CAC GCC ACA CTT CAT GGA CAA AGT ATT GAT GGG TTC TAT GCT GGT      2560
Gly His Ala Thr Leu His Gly Gln Ser Ile Asp Gly Phe Tyr Ala Gly
    650                 655                 660

TCT TCT ATA AGG TAC AGC TGT GAG GTT CTC CAC TAC CTC AGT GGA ACT      2608
Ser Ser Ile Arg Tyr Ser Cys Glu Val Leu His Tyr Leu Ser Gly Thr
665                 670                 675                 680

GAA ACC GTA ACT TGT ACA ACA AAT GGC ACA TGG AGT GCT CCT AAA CCT      2656
Glu Thr Val Thr Cys Thr Thr Asn Gly Thr Trp Ser Ala Pro Lys Pro
                685                 690                 695

CGA TGT ATC AAA GTC ATC ACC TGC CAA AAC CCC CCT GTA CCA TCA TAT      2704
Arg Cys Ile Lys Val Ile Thr Cys Gln Asn Pro Pro Val Pro Ser Tyr
            700                 705                 710

GGT TCT GTG GAA ATC AAA CCC CCA AGT CGG ACA AAC TCG ATA AGT CGT      2752
Gly Ser Val Glu Ile Lys Pro Pro Ser Arg Thr Asn Ser Ile Ser Arg
        715                 720                 725

GTT GGG TCA CCT TTC TTG AGG TTG CCA CGG TTA CCC CTC CCA TTA GCC      2800
Val Gly Ser Pro Phe Leu Arg Leu Pro Arg Leu Pro Leu Pro Leu Ala
    730                 735                 740

AGA GCA GCC AAA CCT CCT CCA AAA CCT AGA TCC TCA CAA CCC TCT ACT      2848
Arg Ala Ala Lys Pro Pro Pro Lys Pro Arg Ser Ser Gln Pro Ser Thr
745                 750                 755                 760

GTG GAC TTG GCT TCT AAA GTT AAA CTA CCT GAA GGT CAT TAC CGG GTA      2896
Val Asp Leu Ala Ser Lys Val Lys Leu Pro Glu Gly His Tyr Arg Val
                765                 770                 775

GGG TCT CGA GCC ATT TAC ACG TGC GAG TCG AGA TAC TAC GAA CTA CTT      2944
Gly Ser Arg Ala Ile Tyr Thr Cys Glu Ser Arg Tyr Tyr Glu Leu Leu
            780                 785                 790

GGA TCT CAA GGC AGA AGA TGT GAC TCT AAT GGA AAC TGG AGT GGT CGG      2992
Gly Ser Gln Gly Arg Arg Cys Asp Ser Asn Gly Asn Trp Ser Gly Arg
        795                 800                 805

CCA GCG AGC TGT ATT CCA GTT TGT GGA CGG TCA GAC TCT CCT CGT TCT      3040
Pro Ala Ser Cys Ile Pro Val Cys Gly Arg Ser Asp Ser Pro Arg Ser
    810                 815                 820

CCT TTT ATC TGG AAT GGG AAT TCT ACA GAA ATA GGT CAG TGG CCG TGG      3088
Pro Phe Ile Trp Asn Gly Asn Ser Thr Glu Ile Gly Gln Trp Pro Trp
825                 830                 835                 840

CAG GCA GGA ATC TCT AGA TGG CTT GCA GAC CAC AAT ATG TGG TTT CTC      3136
Gln Ala Gly Ile Ser Arg Trp Leu Ala Asp His Asn Met Trp Phe Leu
                845                 850                 855
```

```
CAG TGT GGA GGA TCT CTA TTG AAT GAG AAA TGG ATC GTC ACT GCT GCC      3184
Gln Cys Gly Gly Ser Leu Leu Asn Glu Lys Trp Ile Val Thr Ala Ala
            860                 865                 870

CAC TGT GTC ACC TAC TCT GCT ACT GCT GAG ATT ATT GAC CCC AAT CAG      3232
His Cys Val Thr Tyr Ser Ala Thr Ala Glu Ile Ile Asp Pro Asn Gln
        875                 880                 885

TTT AAA ATG TAT CTG GGC AAG TAC TAC CGT GAT GAC AGT AGA GAC GAT      3280
Phe Lys Met Tyr Leu Gly Lys Tyr Tyr Arg Asp Asp Ser Arg Asp Asp
    890                 895                 900

GAC TAT GTA CAA GTA AGA GAG GCT CTT GAG ATC CAC GTG AAT CCT AAC      3328
Asp Tyr Val Gln Val Arg Glu Ala Leu Glu Ile His Val Asn Pro Asn
905                 910                 915                 920

TAC GAC CCC GGC AAT CTC AAC TTT GAC ATA GCC CTA ATT CAA CTG AAA      3376
Tyr Asp Pro Gly Asn Leu Asn Phe Asp Ile Ala Leu Ile Gln Leu Lys
                925                 930                 935

ACT CCT GTT ACT TTG ACA ACA CGA GTC CAA CCA ATC TGT CTG CCT ACT      3424
Thr Pro Val Thr Leu Thr Thr Arg Val Gln Pro Ile Cys Leu Pro Thr
            940                 945                 950

GAC ATC ACA ACA AGA GAA CAC TTG AAG GAG GGA ACA TTA GCA GTG GTG      3472
Asp Ile Thr Thr Arg Glu His Leu Lys Glu Gly Thr Leu Ala Val Val
        955                 960                 965

ACA GGT TGG GGT TTG AAT GAA AAC AAC ACC TAT TCA GAG ACG ATT CAA      3520
Thr Gly Trp Gly Leu Asn Glu Asn Asn Thr Tyr Ser Glu Thr Ile Gln
    970                 975                 980

CAA GCT GTG CTA CCT GTT GTT GCA GCC AGC ACC TGT GAA GAG GGG TAC      3568
Gln Ala Val Leu Pro Val Val Ala Ala Ser Thr Cys Glu Glu Gly Tyr
985                 990                 995                 1000

AAG GAA GCA GAC TTA CCA CTG ACA GTA ACA GAG AAC ATG TTC TGT GCA      3616
Lys Glu Ala Asp Leu Pro Leu Thr Val Thr Glu Asn Met Phe Cys Ala
                1005                1010                1015

GGT TAC AAG AAG GGA CGT TAT GAT GCC TGC AGT GGG GAC AGT GGA GGA      3664
Gly Tyr Lys Lys Gly Arg Tyr Asp Ala Cys Ser Gly Asp Ser Gly Gly
            1020                1025                1030

CCT TTA GTG TTT GCT GAT GAT TCC CGT ACC GAA AGG CGG TGG GTC TTG      3712
Pro Leu Val Phe Ala Asp Asp Ser Arg Thr Glu Arg Arg Trp Val Leu
        1035                1040                1045

GAA GGG ATT GTC AGC TGG GGC AGT CCC AGT GGA TGT GGC AAG GCG AAC      3760
Glu Gly Ile Val Ser Trp Gly Ser Pro Ser Gly Cys Gly Lys Ala Asn
    1050                1055                1060

CAG TAC GGG GGC TTC ACT AAA GTT AAC GTT TTC CTG TCA TGG ATT AGG      3808
Gln Tyr Gly Gly Phe Thr Lys Val Asn Val Phe Leu Ser Trp Ile Arg
1065                1070                1075                1080

CAG TTC ATT TGAAACTGAT CTAAATATTT TAAGCATGGT TATAAACGTC              3857
Gln Phe Ile

TTGTTCCTAT TATTGCTTTA CTGGTTTAAC CCATAAGAAG GTTAACGGGG TAAGGCACAA    3917
```

GGATCATTGT TTCTGTTTGT TTTTACAAAT GGTTCTTTTA GTCAGTGAAT GAGAATAGTA 3977

TCCATTGGAG ACTGTTACCT TTTATTCTAC CTTTTTATAT TACTATGCAA GTATTTGGGA 4037

TATCTTCTAC ACATGAAAAT TCTGTCATTT TACCATAAAT TTGGTTTCTG GTGTGTGTGT 4097

TAAGTCCACC ACTAGAGAAC GATGTAATTT TCAATAGTAC ATGAAATAAA TATAGAACAA 4157

ATCTATTATA AAAAAAAAAA AAAAA 4182

(2) INFORMATION FOR SEQ ID NO:2:

(i) SEQUENCE CHARACTERISTICS:

(A) LENGTH: 1083 amino acids
(B) TYPE: amino acid
(D) TOPOLOGY: linear

(ii) MOLECULE TYPE: protein

(xi) SEQUENCE DESCRIPTION: SEQ ID NO:2:

Met Trp Val Thr Cys Phe Asp Thr Phe Leu Phe Val Cys Glu Ser Ser
1 5 10 15

Val Phe Cys Leu Leu Cys Val Trp Arg Phe Gly Phe Cys Arg Trp Arg
20 25 30

Val Phe Tyr Ser Phe Pro Phe Val Lys Ser Thr Val Val Leu Leu Gln
35 40 45

Cys Tyr His Tyr Ser Leu His Asn Thr Ser Lys Phe Tyr Ser Val Asn
50 55 60

Pro Asp Lys Pro Glu Tyr Ile Leu Ser Gly Leu Val Leu Gly Leu Leu
65 70 75 80

Ala Gln Lys Met Arg Pro Val Gln Ser Lys Gly Val Asp Leu Gly Leu
85 90 95

Cys Asp Glu Thr Arg Phe Glu Cys Lys Cys Gly Asp Pro Gly Tyr Val
100 105 110

Phe Asn Ile Pro Val Lys Gln Cys Thr Tyr Phe Tyr Arg Trp Arg Pro
115 120 125

Tyr Cys Lys Pro Cys Asp Asp Leu Glu Ala Lys Asp Ile Cys Pro Lys
130 135 140

Tyr Lys Arg Cys Gln Glu Cys Lys Ala Gly Leu Asp Ser Cys Val Thr
145 150 155 160

Cys Pro Pro Asn Lys Tyr Gly Thr Trp Cys Ser Gly Glu Cys Gln Cys
165 170 175

Lys Asn Gly Gly Ile Cys Asp Gln Arg Thr Gly Ala Cys Ala Cys Arg
180 185 190

Asp Arg Tyr Glu Gly Val His Cys Glu Ile Leu Lys Gly Cys Pro Leu
195 200 205

Leu Pro Ser Asp Ser Gln Val Gln Glu Val Arg Asn Pro Pro Asp Asn
210 215 220

Pro Gln Thr Ile Asp Tyr Ser Cys Ser Pro Gly Phe Lys Leu Lys Gly
225 230 235 240

Met Ala Arg Ile Ser Cys Leu Pro Asn Gly Gln Trp Ser Asn Phe Pro
245 250 255

Pro Lys Cys Ile Arg Glu Cys Ala Met Val Ser Ser Pro Glu His Gly
260 265 270

Lys Val Asn Ala Leu Ser Gly Asp Met Ile Glu Gly Ala Thr Leu Arg
275 280 285

Phe Ser Cys Asp Ser Pro Tyr Tyr Leu Ile Gly Gln Glu Thr Leu Thr
290 295 300

Cys Gln Gly Asn Gly Gln Trp Asn Gly Gln Ile Pro Gln Cys Lys Asn
305 310 315 320

Leu Val Phe Cys Pro Asp Leu Asp Pro Val Asn His Ala Glu His Lys
325 330 335

Val Lys Ile Gly Val Glu Gln Lys Tyr Gly Gln Phe Pro Gln Gly Thr
340 345 350

Glu Val Thr Tyr Thr Cys Ser Gly Asn Tyr Phe Leu Met Gly Phe Asp
355 360 365

Thr Leu Lys Cys Asn Pro Asp Gly Ser Trp Ser Gly Ser Gln Pro Ser
370 375 380

Cys Val Lys Val Ala Asp Arg Glu Val Asp Cys Asp Ser Lys Ala Val
385 390 395 400

Asp Phe Leu Asp Asp Val Gly Glu Pro Val Arg Ile His Cys Pro Ala
405 410 415

Gly Cys Ser Leu Thr Ala Gly Thr Val Trp Gly Thr Ala Ile Tyr His
420 425 430

Glu Leu Ser Ser Val Cys Arg Ala Ala Ile His Ala Gly Lys Leu Pro
435 440 445

Asn Ser Gly Gly Ala Val His Val Val Asn Asn Gly Pro Tyr Ser Asp
450 455 460

Phe Leu Gly Ser Asp Leu Asn Gly Ile Lys Ser Glu Glu Leu Lys Ser
465 470 475 480

Leu Ala Arg Ser Phe Arg Phe Asp Tyr Val Ser Ser Ser Thr Ala Gly
485 490 495

Lys Ser Gly Cys Pro Asp Gly Trp Phe Glu Val Asp Glu Asn Cys Val
500 505 510

Tyr Val Thr Ser Lys Gln Arg Ala Trp Glu Arg Ala Gln Gly Val Cys
515 520 525

Thr Asn Met Ala Ala Arg Leu Ala Val Leu Asp Lys Asp Val Ile Pro
530 535 540

Asn Ser Leu Thr Glu Thr Leu Arg Gly Lys Gly Leu Thr Thr Thr Trp
545 550 555 560

Ile Gly Leu His Arg Leu Asp Ala Glu Lys Pro Phe Ile Trp Glu Leu
565 570 575

Met Asp Arg Ser Asn Val Val Leu Asn Asp Asn Leu Thr Phe Trp Ala
580 585 590

Ser Gly Glu Pro Gly Asn Glu Thr Asn Cys Val Tyr Met Asp Ile Gln
595 600 605

Asp Gln Leu Gln Ser Val Trp Lys Thr Lys Ser Cys Phe Gln Pro Ser
610 615 620

Ser Phe Ala Cys Met Met Asp Leu Ser Asp Arg Asn Lys Ala Lys Cys
625 630 635 640

Asp Asp Pro Gly Ser Leu Glu Asn Gly His Ala Thr Leu His Gly Gln
645 650 655

Ser Ile Asp Gly Phe Tyr Ala Gly Ser Ser Ile Arg Tyr Ser Cys Glu
660 665 670

Val Leu His Tyr Leu Ser Gly Thr Glu Thr Val Thr Cys Thr Thr Asn
675 680 685

Gly Thr Trp Ser Ala Pro Lys Pro Arg Cys Ile Lys Val Ile Thr Cys
690 695 700

Gln Asn Pro Pro Val Pro Ser Tyr Gly Ser Val Glu Ile Lys Pro Pro
705 710 715 720

Ser Arg Thr Asn Ser Ile Ser Arg Val Gly Ser Pro Phe Leu Arg Leu
725 730 735

Pro Arg Leu Pro Leu Pro Leu Ala Arg Ala Ala Lys Pro Pro Pro Lys
740 745 750

Pro Arg Ser Ser Gln Pro Ser Thr Val Asp Leu Ala Ser Lys Val Lys
755 760 765

Leu Pro Glu Gly His Tyr Arg Val Gly Ser Arg Ala Ile Tyr Thr Cys
770 775 780

Glu Ser Arg Tyr Tyr Glu Leu Leu Gly Ser Gln Gly Arg Arg Cys Asp
785 790 795 800

Ser Asn Gly Asn Trp Ser Gly Arg Pro Ala Ser Cys Ile Pro Val Cys
805 810 815

Gly Arg Ser Asp Ser Pro Arg Ser Pro Phe Ile Trp Asn Gly Asn Ser
820 825 830

```
Thr Glu Ile Gly Gln Trp Pro Trp Gln Ala Gly Ile Ser Arg Trp Leu
        835                 840                 845

Ala Asp His Asn Met Trp Phe Leu Gln Cys Gly Gly Ser Leu Leu Asn
    850                 855                 860

Glu Lys Trp Ile Val Thr Ala Ala His Cys Val Thr Tyr Ser Ala Thr
865                 870                 875                 880

Ala Glu Ile Ile Asp Pro Asn Gln Phe Lys Met Tyr Leu Gly Lys Tyr
                885                 890                 895

Tyr Arg Asp Asp Ser Arg Asp Asp Asp Tyr Val Gln Val Arg Glu Ala
            900                 905                 910

Leu Glu Ile His Val Asn Pro Asn Tyr Asp Pro Gly Asn Leu Asn Phe
        915                 920                 925

Asp Ile Ala Leu Ile Gln Leu Lys Thr Pro Val Thr Leu Thr Thr Arg
    930                 935                 940

Val Gln Pro Ile Cys Leu Pro Thr Asp Ile Thr Thr Arg Glu His Leu
945                 950                 955                 960

Lys Glu Gly Thr Leu Ala Val Val Thr Gly Trp Gly Leu Asn Glu Asn
                965                 970                 975

Asn Thr Tyr Ser Glu Thr Ile Gln Gln Ala Val Leu Pro Val Val Ala
            980                 985                 990

Ala Ser Thr Cys Glu Glu Gly Tyr Lys Glu Ala Asp Leu Pro Leu Thr
        995                 1000                1005

Val Thr Glu Asn Met Phe Cys Ala Gly Tyr Lys Lys Gly Arg Tyr Asp
    1010                1015                1020

Ala Cys Ser Gly Asp Ser Gly Gly Pro Leu Val Phe Ala Asp Asp Ser
1025                1030                1035                1040

Arg Thr Glu Arg Arg Trp Val Leu Glu Gly Ile Val Ser Trp Gly Ser
                1045                1050                1055

Pro Ser Gly Cys Gly Lys Ala Asn Gln Tyr Gly Gly Phe Thr Lys Val
            1060                1065                1070

Asn Val Phe Leu Ser Trp Ile Arg Gln Phe Ile
        1075                1080
```

(2) INFORMATION FOR SEQ ID NO:3:

(i) SEQUENCE CHARACTERISTICS:

(A) LENGTH: 3448 base pairs
(B) TYPE: nucleic acid
(C) STRANDEDNESS: single
(D) TOPOLOGY: both

(ii) MOLECULE TYPE: cDNA

(iii) HYPOTHETICAL: NO

(iv) ANTI-SENSE: NO

(vi) ORIGINAL SOURCE:

(A) ORGANISM: Carcinoscorpius rotundicauda

(ix) FEATURE:

(A) NAME/KEY: CDS
(B) LOCATION: 18..3074

(xi) SEQUENCE DESCRIPTION: SEQ ID NO:3:

```
GTGAAGGTAA CTTAAGT ATG GTC TTA GCG TCG TTT TTG GTG TCT GGT TTA        50
                   Met Val Leu Ala Ser Phe Leu Val Ser Gly Leu
                     1               5                  10

GTT CTA GGG CTA CTA GCC CAA AAA ATG CGC CCA GTT CAG TCC AAA GGA       98
Val Leu Gly Leu Leu Ala Gln Lys Met Arg Pro Val Gln Ser Lys Gly
             15                  20                  25

GTA GAT CTA GGC TTG TGT GAT GAA ACG AGG TTC GAG TGT AAG TGT GGC      146
Val Asp Leu Gly Leu Cys Asp Glu Thr Arg Phe Glu Cys Lys Cys Gly
         30                  35                  40

GAT CCA GGC TAT GTG TTC AAC ATT CCA GTG AAA CAA TGT ACA TAC TTT      194
Asp Pro Gly Tyr Val Phe Asn Ile Pro Val Lys Gln Cys Thr Tyr Phe
     45                  50                  55

TAT CGA TGG AGG CCG TAT TGT AAA CCA TGT GAT GAC CTG GAG GCT AAG      242
Tyr Arg Trp Arg Pro Tyr Cys Lys Pro Cys Asp Asp Leu Glu Ala Lys
 60                  65                  70                  75

GAT ATT TGT CCA AAG TAC AAA CGA TGT CAA GAG TGT AAG GCT GGT CTT      290
Asp Ile Cys Pro Lys Tyr Lys Arg Cys Gln Glu Cys Lys Ala Gly Leu
                 80                  85                  90

GAT AGT TGT GTT ACT TGT CCA CCT AAC AAA TAT GGT ACT TGG TGT AGC      338
Asp Ser Cys Val Thr Cys Pro Pro Asn Lys Tyr Gly Thr Trp Cys Ser
             95                 100                 105

GGT GAA TGT CAG TGT AAG AAT GGA GGT ATC TGT GAC CAG AGG ACA GGA      386
Gly Glu Cys Gln Cys Lys Asn Gly Gly Ile Cys Asp Gln Arg Thr Gly
        110                 115                 120

GCT TGT GCA TGT CGT GAC AGA TAT GAA GGG GTG CAC TGT GAA ATT CTC      434
Ala Cys Ala Cys Arg Asp Arg Tyr Glu Gly Val His Cys Glu Ile Leu
    125                 130                 135

AAA GGT TGT CCT CTT CTT CCA TCG GAT TCT CAG GTT CAG GAA GTC AGA      482
Lys Gly Cys Pro Leu Leu Pro Ser Asp Ser Gln Val Gln Glu Val Arg
140                 145                 150                 155

AAT CCA CCA GAT AAT CCC CAA ACT ATT GAC TAC AGC TGT TCA CCA GGG      530
Asn Pro Pro Asp Asn Pro Gln Thr Ile Asp Tyr Ser Cys Ser Pro Gly
                160                 165                 170

TTC AAG CTT AAG GGT ATG GCA CGA ATT AGC TGT CTC CCA AAT GGA CAG      578
Phe Lys Leu Lys Gly Met Ala Arg Ile Ser Cys Leu Pro Asn Gly Gln
            175                 180                 185
```

TGG AGT AAC TTT CCA CCC AAA TGT ATT CGA GAA TGT GCC ATG GTT TCA 626
Trp Ser Asn Phe Pro Pro Lys Cys Ile Arg Glu Cys Ala Met Val Ser
190 195 200

TCT CCA GAA CAT GGG AAA GTG AAT GCT CTT AGT GGT GAT ATG ATA GAA 674
Ser Pro Glu His Gly Lys Val Asn Ala Leu Ser Gly Asp Met Ile Glu
205 210 215

GGG GCT ACT TTA CGG TTC TCA TGT GAT AGT CCC TAC TAC TTG ATT GGT 722
Gly Ala Thr Leu Arg Phe Ser Cys Asp Ser Pro Tyr Tyr Leu Ile Gly
220 225 230 235

CAA GAA ACA TTA ACC TGT CAG GGT AAT GGT CAG TGG AAT GGA CAG ATA 770
Gln Glu Thr Leu Thr Cys Gln Gly Asn Gly Gln Trp Asn Gly Gln Ile
240 245 250

CCA CAA TGT AAG AAC TTG GTC TTC TGT CCT GAC CTG GAT CCT GTA AAC 818
Pro Gln Cys Lys Asn Leu Val Phe Cys Pro Asp Leu Asp Pro Val Asn
255 260 265

CAT GCT GAA CAC AAG GTT AAA ATT GGT GTG GAA CAA AAA TAT GGT CAG 866
His Ala Glu His Lys Val Lys Ile Gly Val Glu Gln Lys Tyr Gly Gln
270 275 280

TTT CCT CAA GGC ACT GAA GTG ACC TAT ACG TGT TCG GGT AAC TAC TTC 914
Phe Pro Gln Gly Thr Glu Val Thr Tyr Thr Cys Ser Gly Asn Tyr Phe
285 290 295

TTG ATG GGT TTT GAC ACC TTA AAA TGT AAC CCT GAT GGG TCT TGG TCA 962
Leu Met Gly Phe Asp Thr Leu Lys Cys Asn Pro Asp Gly Ser Trp Ser
300 305 310 315

GGA TCA CAG CCA TCC TGT GTT AAA GTG GCA GAC AGA GAG GTC GAC TGT 1010
Gly Ser Gln Pro Ser Cys Val Lys Val Ala Asp Arg Glu Val Asp Cys
320 325 330

GAC AGT AAA GCT GTA GAC TTC TTG GAT GAT GTT GGT GAA CCT GTC AGG 1058
Asp Ser Lys Ala Val Asp Phe Leu Asp Asp Val Gly Glu Pro Val Arg
335 340 345

ATC CAC TGT CCT GCT GGC TGT TCT TTG ACA GCT GGT ACT GTG TGG GGT 1106
Ile His Cys Pro Ala Gly Cys Ser Leu Thr Ala Gly Thr Val Trp Gly
350 355 360

ACA GCC ATA TAC CAT GAA CTT TCC TCA GTG TGT CGT GCA GCC ATC CAT 1154
Thr Ala Ile Tyr His Glu Leu Ser Ser Val Cys Arg Ala Ala Ile His
365 370 375

GCT GGC AAG CTT CCA AAC TCT GGA GGA GCG GTG CAT GTT GTG AAC AAT 1202
Ala Gly Lys Leu Pro Asn Ser Gly Gly Ala Val His Val Val Asn Asn
380 385 390 395

GGC CCC TAC TCG GAC TTT CTG GGT AGT GAC CTG AAT GGG ATA AAA TCG 1250
Gly Pro Tyr Ser Asp Phe Leu Gly Ser Asp Leu Asn Gly Ile Lys Ser
400 405 410

GAA GAG TTG AAG TCT CTT GCC CGG AGT TTC CGA TTC GAT TAT GTC CGT 1298
Glu Glu Leu Lys Ser Leu Ala Arg Ser Phe Arg Phe Asp Tyr Val Arg
415 420 425

```
TCC TCC ACA GCA GGT AAA TCA GGA TGT CCT GAT GGA TGG TTT GAG GTA      1346
Ser Ser Thr Ala Gly Lys Ser Gly Cys Pro Asp Gly Trp Phe Glu Val
        430                 435                 440

GAC GAG AAC TGT GTG TAC GTT ACA TCA AAA CAG AGA GCC TGG GAA AGA      1394
Asp Glu Asn Cys Val Tyr Val Thr Ser Lys Gln Arg Ala Trp Glu Arg
    445                 450                 455

GCT CAA GGT GTG TGT ACC AAT ATG GCT GCT CGT CTT GCT GTG CTG GAC      1442
Ala Gln Gly Val Cys Thr Asn Met Ala Ala Arg Leu Ala Val Leu Asp
460                 465                 470                 475

AAA GAT GTA ATT CCA AAT TCG TTG ACT GAG ACT CTA CGA GGG AAA GGG      1490
Lys Asp Val Ile Pro Asn Ser Leu Thr Glu Thr Leu Arg Gly Lys Gly
                480                 485                 490

TTA ACA ACC ACG TGG ATA GGA TTG CAC AGA CTA GAT GCT GAG AAG CCC      1538
Leu Thr Thr Thr Trp Ile Gly Leu His Arg Leu Asp Ala Glu Lys Pro
            495                 500                 505

TTT ATT TGG GAG TTA ATG GAT CGT AGT AAT GTG GTT CTG AAT GAT AAC      1586
Phe Ile Trp Glu Leu Met Asp Arg Ser Asn Val Val Leu Asn Asp Asn
        510                 515                 520

CTA ACA TTC TGG GCC TCT GGC GAA CCT GGA AAT GAA ACT AAC TGT GTA      1634
Leu Thr Phe Trp Ala Ser Gly Glu Pro Gly Asn Glu Thr Asn Cys Val
    525                 530                 535

TAT ATG GAC ATC CAA GAT CAG TTG CAG TCT GTG TGG AAA ACC AAG TCA      1682
Tyr Met Asp Ile Gln Asp Gln Leu Gln Ser Val Trp Lys Thr Lys Ser
540                 545                 550                 555

TGT TTT CAG CCC TCA AGT TTT GCT TGC ATG ATG GAT CTG TCA GAC AGA      1730
Cys Phe Gln Pro Ser Ser Phe Ala Cys Met Met Asp Leu Ser Asp Arg
                560                 565                 570

AAT AAA GCC AAA TGC GAT GAT CCT GGA TCA CTG GAA AAT GGA CAC GCC      1778
Asn Lys Ala Lys Cys Asp Asp Pro Gly Ser Leu Glu Asn Gly His Ala
            575                 580                 585

ACA CTT CAT GGA CAA AGT ATT GAT GGG TTC TAT GCT GGT TCT TCT ATA      1826
Thr Leu His Gly Gln Ser Ile Asp Gly Phe Tyr Ala Gly Ser Ser Ile
        590                 595                 600

AGG TAC AGC TGT GAG GTT CTC CAC TAC CTC AGT GGA ACT GAA ACC GTA      1874
Arg Tyr Ser Cys Glu Val Leu His Tyr Leu Ser Gly Thr Glu Thr Val
    605                 610                 615

ACT TGT ACA ACA AAT GGC ACA TGG AGT GCT CCT AAA CCT CGA TGT ATC      1922
Thr Cys Thr Thr Asn Gly Thr Trp Ser Ala Pro Lys Pro Arg Cys Ile
620                 625                 630                 635

AAA GTC ATC ACC TGC CAA AAC CCC CCT GTA CCA TCA TAT GGT TCT GTG      1970
Lys Val Ile Thr Cys Gln Asn Pro Pro Val Pro Ser Tyr Gly Ser Val
                640                 645                 650

GAA ATC AAA CCC CCA AGT CGG ACA AAC TCG ATA AGT CGT GTT GGG TCA      2018
Glu Ile Lys Pro Pro Ser Arg Thr Asn Ser Ile Ser Arg Val Gly Ser
            655                 660                 665
```

```
CCT TTC TTG AGG TTG CCA CGG TTA CCC CTC CCA TTA GCT AGA GCA GCC     2066
Pro Phe Leu Arg Leu Pro Arg Leu Pro Leu Pro Leu Ala Arg Ala Ala
        670                 675                 680

AAA CCT CCT CCA AAA CCT AGA TCC TCA CAA CCC TCT ACT GTG GAC TTG     2114
Lys Pro Pro Pro Lys Pro Arg Ser Ser Gln Pro Ser Thr Val Asp Leu
    685                 690                 695

GCT TCT AAA GTT AAA CTA CCT GAA GGT CAT TAC CGG GTA GGG TCT CGA     2162
Ala Ser Lys Val Lys Leu Pro Glu Gly His Tyr Arg Val Gly Ser Arg
700                 705                 710                 715

GCC ATC TAC ACG TGC GAG TCG AGA TAC TAC GAA CTA CTT GGA TCT CAA     2210
Ala Ile Tyr Thr Cys Glu Ser Arg Tyr Tyr Glu Leu Leu Gly Ser Gln
                720                 725                 730

GGC AGA AGA TGT GAC TCT AAT GGA AAC TGG AGT GGT CGG CCA GCG AGC     2258
Gly Arg Arg Cys Asp Ser Asn Gly Asn Trp Ser Gly Arg Pro Ala Ser
            735                 740                 745

TGT ATT CCA GTT TGT GGA CGG TCA GAC TCT CCT CGT TCT CCT TTT ATC     2306
Cys Ile Pro Val Cys Gly Arg Ser Asp Ser Pro Arg Ser Pro Phe Ile
        750                 755                 760

TGG AAT GGG AAT TCT ACA GAA ATA GGT CAG TGG CCG TGG CAG GCA GGA     2354
Trp Asn Gly Asn Ser Thr Glu Ile Gly Gln Trp Pro Trp Gln Ala Gly
    765                 770                 775

ATC TCT AGA TGG CTT GCA GAC CAC AAT ATG TGG TTT CTC CAG TGT GGA     2402
Ile Ser Arg Trp Leu Ala Asp His Asn Met Trp Phe Leu Gln Cys Gly
780                 785                 790                 795

GGA TCT CTA TTG AAT GAG AAA TGG ATC GTC ACT GCT GCC CAC TGT GTC     2450
Gly Ser Leu Leu Asn Glu Lys Trp Ile Val Thr Ala Ala His Cys Val
                800                 805                 810

ACC TAC TCT GCT ACT GCT GAG ATT ATT GAC CCC AAT CAG TTT AAA ATG     2498
Thr Tyr Ser Ala Thr Ala Glu Ile Ile Asp Pro Asn Gln Phe Lys Met
            815                 820                 825

TAT CTG GGC AAG TAC TAC CGT GAT GAC AGT AGA GAC GAT GAC TAT GTA     2546
Tyr Leu Gly Lys Tyr Tyr Arg Asp Asp Ser Arg Asp Asp Asp Tyr Val
        830                 835                 840

CAA GTA AGA GAG GCT CTT GAG ATC CAC GTG AAT CCT AAC TAC GAC CCC     2594
Gln Val Arg Glu Ala Leu Glu Ile His Val Asn Pro Asn Tyr Asp Pro
    845                 850                 855

GGC AAT CTC AAC TTT GAC ATA GCC CTA ATT CAA CTG AAA ACT CCT GTT     2642
Gly Asn Leu Asn Phe Asp Ile Ala Leu Ile Gln Leu Lys Thr Pro Val
860                 865                 870                 875

ACT TTG ACA ACA CGA GTC CAA CCA ATC TGT CTG CCT ACT GAC ATC ACA     2690
Thr Leu Thr Thr Arg Val Gln Pro Ile Cys Leu Pro Thr Asp Ile Thr
                880                 885                 890

ACA AGA GAA CAC TTG AAG GAG GGA ACA TTA GCA GTG GTG ACA GGT TGG     2738
Thr Arg Glu His Leu Lys Glu Gly Thr Leu Ala Val Val Thr Gly Trp
            895                 900                 905
```

```
GGT TTG AAT GAA AAC AAC ACC TAT TCA GAG ACG ATT CAA CAA GCT GTG      2786
Gly Leu Asn Glu Asn Asn Thr Tyr Ser Glu Thr Ile Gln Gln Ala Val
    910                     915                     920

CTA CCT GTT GTT GCA GCC AGC ACC TGT GAA GAG GGG TAC AAG GAA GCA      2834
Leu Pro Val Val Ala Ala Ser Thr Cys Glu Glu Gly Tyr Lys Glu Ala
    925                     930                     935

GAC TTA CCA CTG ACA GTA ACA GAG AAC ATG TTC TGT GCA GGT TAC AAG      2882
Asp Leu Pro Leu Thr Val Thr Glu Asn Met Phe Cys Ala Gly Tyr Lys
940                     945                     950                     955

AAG GGA CGT TAT GAT GCC TGC AGT GGG GAC AGT GGA GGA CCT TTA GTG      2930
Lys Gly Arg Tyr Asp Ala Cys Ser Gly Asp Ser Gly Gly Pro Leu Val
                960                     965                     970

TTT GCT GAT GAT TCC CGT ACC GAA AGG CGG TGG GTC TTG GAA GGG ATT      2978
Phe Ala Asp Asp Ser Arg Thr Glu Arg Arg Trp Val Leu Glu Gly Ile
            975                     980                     985

GTC AGC TGG GGC AGT CCC AGT GGA TGT GGC AAG GCG AAC CAG TAC GGG      3026
Val Ser Trp Gly Ser Pro Ser Gly Cys Gly Lys Ala Asn Gln Tyr Gly
        990                     995                     1000

GGC TTC ACT AAA GTT AAC GTT TTC CTG TCA TGG ATT AGG CAG TTC ATT      3074
Gly Phe Thr Lys Val Asn Val Phe Leu Ser Trp Ile Arg Gln Phe Ile
    1005                    1010                    1015

TGAAACTGAT CTAAATATTT TAAGCATGGT TATAAACGTC TTGTTTCCTA TTATTGCTTT    3134

ACTAGTTTAA CCCATAAGAA GGTTAACTGG GTAAGGCACA AGGATCATTG TTTCTGTTTG    3194

TTTTTACAAA TGGTTATTTT AGTCAGTGAA TGAGAATAGT ATCCATTGAA GACTGTTACC    3254

TTTTATTCTA CCTTTTTATA TTACTATGTA AGTATTTGGG ATATCTTCTA CACATGAAAA    3314

TTCTGTCATT TTACCATAAA TTTGGTTTCT GGTGTGTGCT AAGTCCACCA GTAGAGAACG    3374

ATGTAATTTT CACTAGCACA TGAAATAAAT ATAGAACAAA TCTATTATAA ACTACCTTAA    3434

AAAAAAAAAA AAAA                                                      3448
```

(2) INFORMATION FOR SEQ ID NO:4:

(i) SEQUENCE CHARACTERISTICS:

(A) LENGTH: 1019 amino acids
(B) TYPE: amino acid
(D) TOPOLOGY: linear

(ii) MOLECULE TYPE: protein

(xi) SEQUENCE DESCRIPTION: SEQ ID NO:4:

```
Met Val Leu Ala Ser Phe Leu Val Ser Gly Leu Val Leu Gly Leu Leu
1               5                   10                  15

Ala Gln Lys Met Arg Pro Val Gln Ser Lys Gly Val Asp Leu Gly Leu
            20                  25                  30
```

```
Cys Asp Glu Thr Arg Phe Glu Cys Lys Cys Gly Asp Pro Gly Tyr Val
        35                  40                  45

Phe Asn Ile Pro Val Lys Gln Cys Thr Tyr Phe Tyr Arg Trp Arg Pro
    50                  55                  60

Tyr Cys Lys Pro Cys Asp Asp Leu Glu Ala Lys Asp Ile Cys Pro Lys
65                  70                  75                  80

Tyr Lys Arg Cys Gln Glu Cys Lys Ala Gly Leu Asp Ser Cys Val Thr
                85                  90                  95

Cys Pro Pro Asn Lys Tyr Gly Thr Trp Cys Ser Gly Glu Cys Gln Cys
            100                 105                 110

Lys Asn Gly Gly Ile Cys Asp Gln Arg Thr Gly Ala Cys Ala Cys Arg
        115                 120                 125

Asp Arg Tyr Glu Gly Val His Cys Glu Ile Leu Lys Gly Cys Pro Leu
    130                 135                 140

Leu Pro Ser Asp Ser Gln Val Gln Glu Val Arg Asn Pro Pro Asp Asn
145                 150                 155                 160

Pro Gln Thr Ile Asp Tyr Ser Cys Ser Pro Gly Phe Lys Leu Lys Gly
                165                 170                 175

Met Ala Arg Ile Ser Cys Leu Pro Asn Gly Gln Trp Ser Asn Phe Pro
            180                 185                 190

Pro Lys Cys Ile Arg Glu Cys Ala Met Val Ser Ser Pro Glu His Gly
        195                 200                 205

Lys Val Asn Ala Leu Ser Gly Asp Met Ile Glu Gly Ala Thr Leu Arg
    210                 215                 220

Phe Ser Cys Asp Ser Pro Tyr Tyr Leu Ile Gly Gln Glu Thr Leu Thr
225                 230                 235                 240

Cys Gln Gly Asn Gly Gln Trp Asn Gly Gln Ile Pro Gln Cys Lys Asn
                245                 250                 255

Leu Val Phe Cys Pro Asp Leu Asp Pro Val Asn His Ala Glu His Lys
            260                 265                 270

Val Lys Ile Gly Val Glu Gln Lys Tyr Gly Gln Phe Pro Gln Gly Thr
        275                 280                 285

Glu Val Thr Tyr Thr Cys Ser Gly Asn Tyr Phe Leu Met Gly Phe Asp
    290                 295                 300

Thr Leu Lys Cys Asn Pro Asp Gly Ser Trp Ser Gly Ser Gln Pro Ser
305                 310                 315                 320

Cys Val Lys Val Ala Asp Arg Glu Val Asp Cys Asp Ser Lys Ala Val
                325                 330                 335

Asp Phe Leu Asp Asp Val Gly Glu Pro Val Arg Ile His Cys Pro Ala
            340                 345                 350
```

```
Gly Cys Ser Leu Thr Ala Gly Thr Val Trp Gly Thr Ala Ile Tyr His
        355                 360                 365

Glu Leu Ser Ser Val Cys Arg Ala Ala Ile His Ala Gly Lys Leu Pro
    370                 375                 380

Asn Ser Gly Gly Ala Val His Val Val Asn Asn Gly Pro Tyr Ser Asp
385                 390                 395                 400

Phe Leu Gly Ser Asp Leu Asn Gly Ile Lys Ser Glu Glu Leu Lys Ser
                405                 410                 415

Leu Ala Arg Ser Phe Arg Phe Asp Tyr Val Arg Ser Ser Thr Ala Gly
            420                 425                 430

Lys Ser Gly Cys Pro Asp Gly Trp Phe Glu Val Asp Glu Asn Cys Val
        435                 440                 445

Tyr Val Thr Ser Lys Gln Arg Ala Trp Glu Arg Ala Gln Gly Val Cys
    450                 455                 460

Thr Asn Met Ala Ala Arg Leu Ala Val Leu Asp Lys Asp Val Ile Pro
465                 470                 475                 480

Asn Ser Leu Thr Glu Thr Leu Arg Gly Lys Gly Leu Thr Thr Thr Trp
                485                 490                 495

Ile Gly Leu His Arg Leu Asp Ala Glu Lys Pro Phe Ile Trp Glu Leu
            500                 505                 510

Met Asp Arg Ser Asn Val Val Leu Asn Asp Asn Leu Thr Phe Trp Ala
        515                 520                 525

Ser Gly Glu Pro Gly Asn Glu Thr Asn Cys Val Tyr Met Asp Ile Gln
    530                 535                 540

Asp Gln Leu Gln Ser Val Trp Lys Thr Lys Ser Cys Phe Gln Pro Ser
545                 550                 555                 560

Ser Phe Ala Cys Met Met Asp Leu Ser Asp Arg Asn Lys Ala Lys Cys
                565                 570                 575

Asp Asp Pro Gly Ser Leu Glu Asn Gly His Ala Thr Leu His Gly Gln
            580                 585                 590

Ser Ile Asp Gly Phe Tyr Ala Gly Ser Ser Ile Arg Tyr Ser Cys Glu
        595                 600                 605

Val Leu His Tyr Leu Ser Gly Thr Glu Thr Val Thr Cys Thr Thr Asn
    610                 615                 620

Gly Thr Trp Ser Ala Pro Lys Pro Arg Cys Ile Lys Val Ile Thr Cys
625                 630                 635                 640

Gln Asn Pro Pro Val Pro Ser Tyr Gly Ser Val Glu Ile Lys Pro Pro
                645                 650                 655

Ser Arg Thr Asn Ser Ile Ser Arg Val Gly Ser Pro Phe Leu Arg Leu
            660                 665                 670
```

```
Pro Arg Leu Pro Leu Pro Leu Ala Arg Ala Ala Lys Pro Pro Pro Lys
    675                 680                 685

Pro Arg Ser Ser Gln Pro Ser Thr Val Asp Leu Ala Ser Lys Val Lys
690                 695                 700

Leu Pro Glu Gly His Tyr Arg Val Gly Ser Arg Ala Ile Tyr Thr Cys
705                 710                 715                 720

Glu Ser Arg Tyr Tyr Glu Leu Leu Gly Ser Gln Gly Arg Arg Cys Asp
                725                 730                 735

Ser Asn Gly Asn Trp Ser Gly Arg Pro Ala Ser Cys Ile Pro Val Cys
            740                 745                 750

Gly Arg Ser Asp Ser Pro Arg Ser Pro Phe Ile Trp Asn Gly Asn Ser
        755                 760                 765

Thr Glu Ile Gly Gln Trp Pro Trp Gln Ala Gly Ile Ser Arg Trp Leu
    770                 775                 780

Ala Asp His Asn Met Trp Phe Leu Gln Cys Gly Gly Ser Leu Leu Asn
785                 790                 795                 800

Glu Lys Trp Ile Val Thr Ala Ala His Cys Val Thr Tyr Ser Ala Thr
                805                 810                 815

Ala Glu Ile Ile Asp Pro Asn Gln Phe Lys Met Tyr Leu Gly Lys Tyr
            820                 825                 830

Tyr Arg Asp Asp Ser Arg Asp Asp Asp Tyr Val Gln Val Arg Glu Ala
        835                 840                 845

Leu Glu Ile His Val Asn Pro Asn Tyr Asp Pro Gly Asn Leu Asn Phe
    850                 855                 860

Asp Ile Ala Leu Ile Gln Leu Lys Thr Pro Val Thr Leu Thr Thr Arg
865                 870                 875                 880

Val Gln Pro Ile Cys Leu Pro Thr Asp Ile Thr Thr Arg Glu His Leu
                885                 890                 895

Lys Glu Gly Thr Leu Ala Val Val Thr Gly Trp Gly Leu Asn Glu Asn
            900                 905                 910

Asn Thr Tyr Ser Glu Thr Ile Gln Gln Ala Val Leu Pro Val Val Ala
        915                 920                 925

Ala Ser Thr Cys Glu Glu Gly Tyr Lys Glu Ala Asp Leu Pro Leu Thr
    930                 935                 940

Val Thr Glu Asn Met Phe Cys Ala Gly Tyr Lys Lys Gly Arg Tyr Asp
945                 950                 955                 960

Ala Cys Ser Gly Asp Ser Gly Gly Pro Leu Val Phe Ala Asp Asp Ser
                965                 970                 975

Arg Thr Glu Arg Arg Trp Val Leu Glu Gly Ile Val Ser Trp Gly Ser
            980                 985                 990
```

Pro Ser Gly Cys Gly Lys Ala Asn Gln Tyr Gly Gly Phe Thr Lys Val
995 1000 1005

Asn Val Phe Leu Ser Trp Ile Arg Gln Phe Ile
1010 1015